(19) Europäisches Patentamt
European Patent Office
Office européen des brevets

(11) Publication number: **0 357 226 A1**

(12) **EUROPEAN PATENT APPLICATION**

(21) Application number: **89307708.1**

(22) Date of filing: **28.07.89**

(51) Int. Cl.[5]: **C07H 17/02 , A61K 31/70 , C12P 19/60 , //(C12P19/60, C12R1:03)**

The microorganism(s) has (have) been deposited with American Type Culture Collection under number ATCC 53714.

(30) Priority: **03.08.88 US 227963**
**03.08.88 US 227968**
**03.08.88 US 227964**
**03.08.88 US 227951**

(43) Date of publication of application:
**07.03.90 Bulletin 90/10**

(84) Designated Contracting States:
**ES GR**

(71) Applicant: **SCHERING CORPORATION**
**2000 Galloping Hill Road**
**Kenilworth New Jersey 07033(US)**

(72) Inventor: **Hedge, Vinod R.**
**11 Sanders Road**
**Rockaway New Jersey 07866(US)**
Inventor: **Horan, Ann C.**
**144 Mountain Avenue**
**Summit New Jersey 07901(US)**
Inventor: **King, Arthur H.**
**23 Monroe Avenue**
**East Orange New Jersey 07017(US)**
Inventor: **Gentile, Frank A.**
**22 Schuyler Road**
**Wayne New Jersey 07470(US)**
Inventor: **Patel, Mahesh G.**
**42 Brentwood Drive**
**Verona New Jersey 07044(US)**
Inventor: **Wagman, Gerald H.**
**17 Crommelin Court**
**East Brunswick New Jersey 08816(US)**
Inventor: **Gunnarsson, Ingrid-Agneta**
**147 Kings Highway**
**Hackettstown New Jersey 07840(US)**
Inventor: **Truumees, Imbi**
**40 Gilmore Avenue**
**Cresskill New Jersey 07626(US)**
Inventor: **Cooper, Raymond**
**15 Vauxhall Road**
**East Brunswick New Jersey 08816(US)**
Inventor: **Yarborough, Raymond F.**
**645 Union Street**
**Orange New Jersey 07050(US)**

(74) Representative: **Ritter, Stephen David et al**
**Mathys & Squire 10 Fleet Street**
**London EC4Y 1AY(GB)**

(54) **Macrolactam antimicrobial compounds.**

(57) Compounds having the formula:

EP 0 357 226 A1

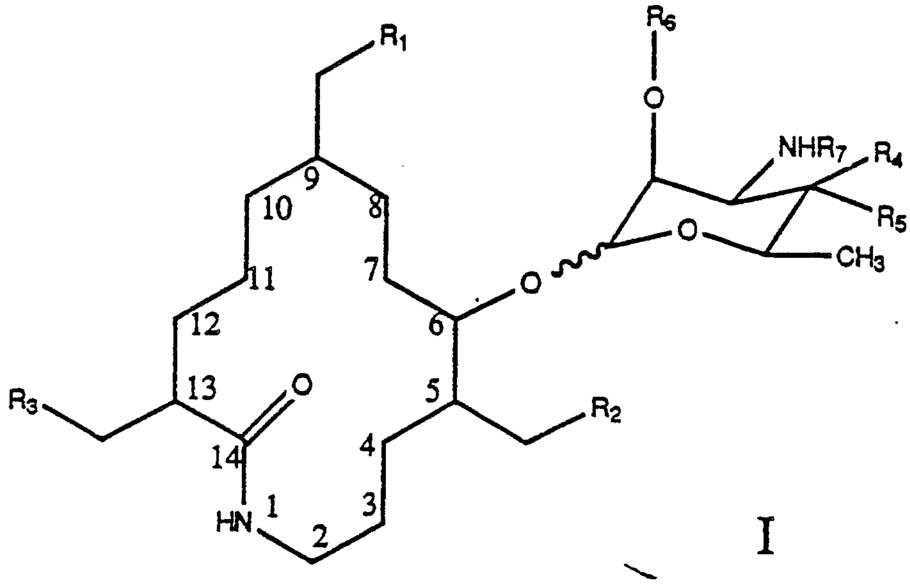

I

wherein either

(a) $R_1$, $R_2$, and $R_3$ are methyl,

$R_4$ is hydrogen,

$R_5$ is hydroxy,

$R_6$ is

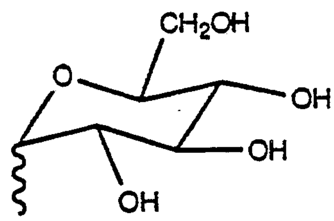

;

and

$R_7$ is hydrogen;

<u>or</u>

(b) $R_1$, $R_2$, ad $R_3$ are methyl

$R_4$ is hydrogen,

$R_6$ is

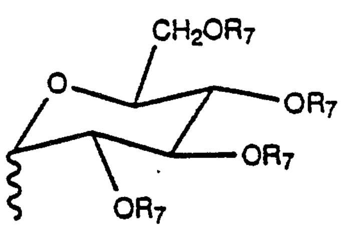

;

and

$R_5$ and each $R_7$ are independently a straight or branched chain ($C_1$-$C_{22}$) alkanoyl group, a straight or branched chain ($C_3$-$C_{22}$) alkenoyl group or hydrogen, with the proviso that $R_5$ or at least one $R_7$ is not hydrogen;

or

(c) $R_1$, $R_2$, and $R_3$ are methyl,
$R_4$ is hydroxy, and
$R_5$, $R_6$, $R_7$ are hydrogen;

or

(d) $R_1$, $R_2$, and $R_3$ are hydrogen, or
$R_1$ and $R_2$ are hydrogen and $R_3$ is methyl,
or $R_1$ is hydrogen and $R_2$ and $R_3$ are methyl,
$R_5$ is hydroxy, and
$R_4$, $R_6$ and $R_7$ are hydrogen;

or

(e) $R_1$ is hydrogen,
$R_2$ and $R_3$ are methyl
$R_4$ is hydroxy, and
$R_5$, $R_6$, and $R_7$ are hydrogen or a pharmaceutically acceptable salt thereof.

The compounds are obtained by cultivating various bacteria as disclosed herein. This invention also contemplates treatment of microbial infections with pharmaceutical compositions containing the compounds, the compositions per se, a biologically pure culture of the bacteria that produces the compounds, and antimicrobial complexes produced by the bacteria.

## MACROLACTAM ANTIMICROBIAL COMPOUNDS

This invention relates to a macrolactam antimicrobial compounds.

### SUMMARY OF THE INVENTION

A first aspect of the invention may be summarized as a compound having the formula:

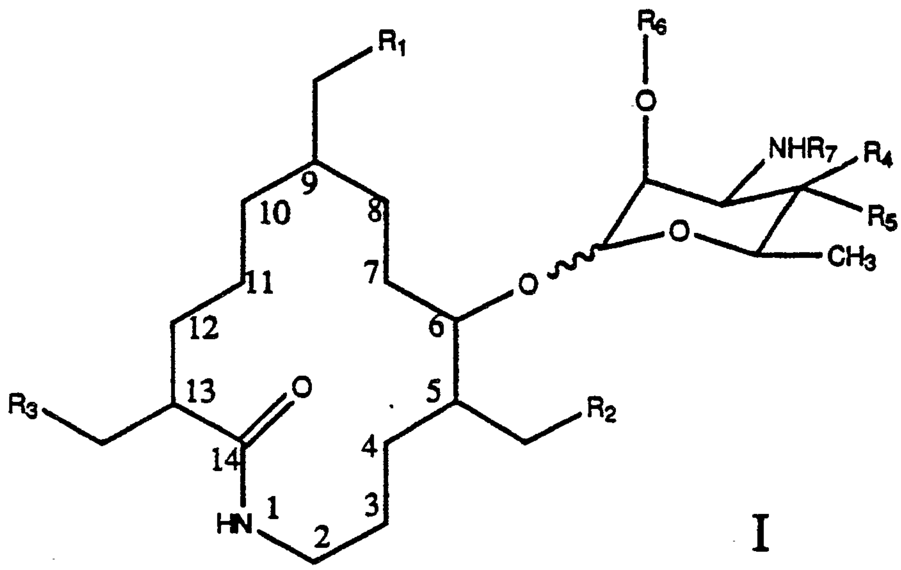

I

wherein either

(a) $R_1$, $R_2$, and $R_3$ are methyl,
$R_4$ is hydrogen,
$R_5$ is hydroxy,
$R_6$ is

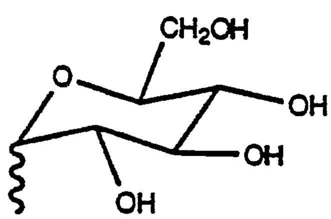

;

and
$R_7$ is hydrogen;
or

(b) $R_1$, $R_2$, and $R_3$ are methyl
$R_4$ is hydrogen,
$R_6$ is

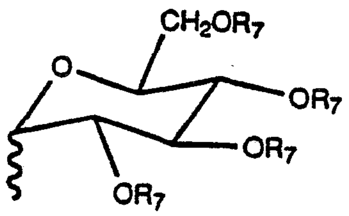

;

and

$R_5$ and each $R_7$ are independently a straight or branched chain ($C_1$-$C_{22}$) alkanoyl group, a straight or branched chain ($C_3$-$C_{22}$) alkenoyl group or hydrogen, with the proviso that $R_5$ or at least one $R_7$ is not hydrogen;

or

(c) $R_1$, $R_2$, and $R_3$ are methyl,
$R_4$ is hydroxy, and
$R_5$, $R_6$, $R_7$ are hydrogen;

or

(d) $R_1$, $R_2$, and $R_3$ are hydrogen, or
$R_1$ and $R_2$ are hydrogen and $R_3$ is methyl,
or $R_1$ is hydrogen and $R_2$ and $R_3$ are methyl,
$R_5$ is hydroxy, and
$R_4$, $R_6$ and $R_7$ are hydrogen;

or

(e) $R_1$ is hydrogen,
$R_2$ and $R_3$ are methyl
$R_4$ is hydroxy, and
$R_5$, $R_6$, and $R_7$ are hydrogen or a pharmaceutically acceptable salt thereof.

Another aspect of the invention comprises a pharmaceutical composition comprising an antimicrobially effective amount of the compound of Formula I and a pharmaceutically acceptable carrier or diluent.

A third aspect of the invention comprises a method for making a pharmaceutical composition for treating microbial infection comprising admixing a compound of Formula I with a pharmaceutically acceptable carrier or diluent.

Another aspect of the invnetion comprises a biologically pure culture of the microorganism Actinomadura fulva subsp. indica, SCC 1840 having the identifying characteristics of ATCC 53714 said culture being capable of producing the antimicrobial complex in a recoverable quantity upon fermentation, under aerobic conditions in an aqueous medium containing assimilable sources of nitrogen and carbon.

Another aspect of the invention comprises antimicrobial complex 530 produced by cultivating a strain of Actinomadura fulva subsp. indica having the identifying characteristics of ATCC 53714 in a pH and temperature controlled aqueous nutrient medium containing assimilable sources of nitrogen and carbon, under controlled submerged aerobic conditions until a composition of matter having substantial antimicrobial activity is produced.

Another aspect of the invention comprises a biologically pure culture of the microorganism Actinomadura vulgaris subsp. vulgaris, SCC 1776 having the identifying characteristics of ATCC 53748 said culture being capable of producing the antimicrobial complex in a recoverable quantity upon fermentation, under aerobic conditions in an aqueous medium containing assimilable sources of nitrogen and carbon.

Another aspect of the invention comprises antimicrobial complex 517 produced by cultivating a strain of Actinomadura vulgaris subsp. vulgaris having the identifying characteristics of ATCC 53748 in a pH and temperature controlled aqueous nutrient medium containing assimilable sources of nitrogen and carbon, under controlled submerged aerobic conditions until a composition of matter having substantial antimicrobial activity is produced.

Another aspect of the invention comprises a biologically pure culture of the microorganism Actinomadura fulva subsp. uruguayensis SCC 778 having the identifying characteristics of ATCC 53713 said culture being capable of producing the antimicrobial complex in a recoverable quantity upon fermentation, under aerobic conditions in an aqueous medium containing assimilable sources of nitrogen and carbon.

Another aspect of the invention comprises antimicrobial complex 510 produced by cultivating a strain of Actinomadura fulva subsp. uruguayensis having the identifying characteristics of ATCC 53713 in a pH and temperature controlled aqueous nutrient medium containing assimilable sources of nitrogen and carbon,

under controlled submerged aerobic conditions until a composition of matter having substantial antimicrobial activity is produced.

Another aspect of the invention comprises a biologically pure culture of the microorganism Actinomadura vulgaris subsp. lanata, SCC 1777 having the identifying characteristics of ATCC 53715 said culture being capable of producing the antimicrobial complex in a recoverable quantity upon fermentation, under aerobic conditions in an aqueous medium containing assimilable sources of nitrogen and carbon.

Another aspect of the invention comprises antimicrobial complex 515 produced by cultivating a strain of Actinomadura vulgaris subsp. lanata having the identifying characteristics of ATCC 53715 in a pH and temperature controlled aqueous nutrient medium containing assimilable sources of nitrogen and carbon, under controlled submerged aerobic conditions until a composition of matter having substantial antimicrobial activity is produced.

Another aspect of the invention comprises a method for making a compound of formula I wherein either

(a) to produce a compound having the substituents (a) cultivating a microorganism of the species Actinomadura fulva subsp. indica in an aqueous nutrient media;

(b) to produce a compound having substituents (b) introducing at least one $C_1$-$C_{22}$ alkanoyl and/or $C_3$-$C_{22}$ alkenoyl group to a compound having substituents (a) of this claim by conventinal means;

(c) to produce a compound having the substituents (c), cultivating a microorganism of the species Actinomadura vulgaris subsp. vulgaris in an aqueous nutrient media;

(d) to produce a compound having the substituents (d), cultivating a microorganism of the species Actinomadura fulva subsp. uruguayensis in an aqueous nutrient media; or

(e) to produce a compound having the substituents (e), cultivating a microorganism of the species Actinomadura vulgaris subsp. lanata in an aqueous nutrient media;.

The above process (a), (b), (c), (d), or (e) being followed if necessary or desired by

(i) isolating the so-obtained compound of formula I and/or

(ii) converting the so-obtained compound of formula I to a pharmaceutically acceptable salt.

## DETAILED DESCRIPTION

Each aspect of formula I, (a), (b), (c), (d), and (e), will now be discussed separetly in detail.

## COMPOUNDS OF FORMULA I HAVING SUBSTITUENTS (a) AND (b):

(hereinafter Formulas I(a) and I(b))

## THE MICROORGANISM

The microorganism used for the production of antimicrobial complex 530 and the compound represented by formula I(a) is a biologically pure culture of Actinomadura fulva subsp. indica subsp. nov. SCC 1840, ATCC 53714.

A viable culture of this microorganism has been deposited in the collection of the American Type Culture Collection (ATCC) in Rockville, Md., where it has been assigned accession number ATCC 53714. The deposit was made on January 25, 1988.

The microorganism was isolated from a sample of soil collected in India. It had been characterized and found to have the microscopic, macroscopic, and whole cell hydrolysis properties of the genus Actinomadura.

## DESCRIPTION OF THE PRODUCING STRAIN: ACTINOMADURA FULVA SUBSP. INDICA SCC 1840, ATCC 53714

Source material for the following taxonomic evaluations was a frozen preparation of a pure culture of Actinomadura fulva subsp. indica SCC 1840, ATCC 53714. Inoculum for the biochemical and physiological tests was prepared according to the procedures of Horan and Brodsky [Horan and Brodsky, Int. J. Syst. Bacteriol., Vol. 32, pp. 195-200 (1982)]. The incubation temperature for the biochemical and physiological tests was 30° C. Readings of the results were made at various times up to 21 days for the plate media. Most of the tubed media were read at various times up to 28 days. The tests for decomposition of urea, allantoin and hippurate, as well as the tests for the reduction of nitrates were read for six weeks.

## MORPHOLOGY

Morphological observations of the producing strain of the microorganism of this invention were made on plates of water agar, AV-agar [Nonomura and Ohara, J. Ferment. Technol., Vol. 47, pp. 463-469 (1966)] or modified inorganic salts-starch agar [Difco inorganic salts-starch agar (ISP-4), 12 g; Difco Bacto agar, 15 g; distilled water, 800 ml; soil extract 200 ml; thiamine HCL, 0.5 mg; riboflavin, 0.5 mg; niacin, 0.5 mg; pyridoxine HCL, 0.5 mg; inositol, 0.5 mg; calcium pantothenate, 0.5 mg; p-aminobenzoic acid, 0.5 mg; biotin, 0.25 mg]. Plates were incubated at 30° C and observed for 4 to 6 weeks.

The producing strain of the microorganism of this invention is a gram positive, filamentous organism that forms a well-developed substrate mycelium with non-fragmenting, moderately branching hyphae which are approximately 0.4 µm to 0.8 µm in diameter. There are no spores on the substrate mycelium.

The aerial hyphae, approximately 0.4 µm to 0.9 µm in diameter, bear chains of smooth-walled spores. The spores are round to ovoid and approximately 0.9 t 1.5 µm in diameter. The spore chains contain approximately 6 to 33 spores per chain and are arranged in tightly appressed spirals forming pseudosporangia. The pseudosporangia are approximately 2 to 7 µm in diameter. No motile elements were observed in either the substrate or aerial mycelium.

## CHEMOTAXONOMY

Purified cell wall preparations of the producing strain of this invention were analyzed by the method of Becker [Becker et. al., Appl, Microbiol., Vol. 12, pp. 421-423 (1964)] and shown to contain the mesoisomer of 2,6-diaminopimelic acid, alanine, glutamic acid, glucosamine, muramic acid and traces of mannose. Whole-cell hydroysates were analyzed by the method of Lechevalier [Lechevalier, M.P., J. Lab. Clin. Med., Vol. 71, pp. 934-944 (1968)] and shown to contain glucose, mannose, madurose, ribose, a trace of galaotose and a trace of rhamnose. The phospholipids present are diphosphatidylglycerol, phosphatidylinositol, phosphatidylethanolamine, and unknown glucosamine containing phospholipids. Thus, the producing strain of the microorganism of this invention has a type III cell wall with a type B whole-cell sugar pattern and a type P IV phospholipid composition [Lechevalier et. al., Biochem. System. Ecol., Vol. 5, pp. 249-260 (1977)], typical of actinomadurae.

## PHYSIOLOGICAL AND BIOCHEMICAL CHARACTERISTICS

The procedures used to obtain the captional characteristics were those cited by Gordon [Gordon, R.E., J. Gen. Microbiol., Vol. 45, pp. 355-364 (1966)], Luedemann and Brodsky [Luedemann and Brodsky, "Antimicrob. Agents Chemother." pp 47-52 (1965)] and Horan and Brodsky [Horan and Brodsky, Int. J. Syst. Bacteriol., Vol. 32, pp. 195-200 (1982)]. The producing strain of the microorganism of this invention, SCC 1840, produces acid from adonitol, D-arabinose, L-arabinose, D-fructose, L-fucose, D-galactose, glucose, glycerol, i-inositol, maltose, D-mannitol, D-mannose, α-D-melibiose, α-methyl-d-glucoside, β-methyl-D-glucopyranoside, α-L-rhamnose, D-ribose, sucrose, D-trehalose and D-xylose but not from dulcitol, i-erythritol, D-melizitose, or D-sorbitol. Adenine, hypoxanthine, L-tyrosine, elastin, casein and hippurate are hydrolyzed but guanine, xanthine, xylan and chitin are not. Gelatin is both hydrolyzed and liquified. Starch hydrolysis is negative. Urease is formed; allantoinase is not. Nitrate is reduced to nitrite. Melanin and hydrogen sulfide are not formed. Growth does not occur at 10° C or at 45° C. Growth is fair at 40° C. The microorganism of this invention, SCC 1840, grows poorly at 4% NaCl; good growth occurs at 3% NaCl.

Acetate, butyrate, lactate, pyruvate and succinate are utilized; but benzoate, formate oxalate and tartrate are not.

The producing strain of the microorganisms of the present invention grows in the presence of 50 µg/ml of kanamycin, streptomycin, novobiocin, rifamycin, erythromycin, penicillin G, cephalothin, tetracycline, cycloserine and spectinomycin and in the present of 10 mcg/ml of neomycin. Growth is poor in the presence of 50 mcg/ml of sisomicin, gentamicin and clindamycin.

DESCRIPTION OF A. FULVA SUBSP. INDICA ON VARIOUS MEDIA

All plates were incubated at 30° C and observed at intervals up to 28 days. The common names for the colors were choosen after comparison with color chips from the ISCC-NBS centroid color charts, the "Color Harmony Manual", Ed. 4 (Container Corp. America, 1958), or the "Methuen Handbook of Color" (Eyre Methuen, London, 1981). On all media tested, the substrate mycelium of the microorganism of this invention, SCC 1840, was off-white, yellow-brown or brown. The aerial mycelium was white, yellowish white or ivory. Yellow-brown diffusible pigments were formed. Characteristics are presented in Table 1.

On the basis of its morphological and chemotaxonomic characteristics, the producing strain of of this invention, SCC 1840, was placed in the genus Actinomadura. The description of the microorganism of this invention, SCC 1840, was compared to the descriptions of Actinomadura species found on the approved lists of bacterial names or in the patent literature. The microorganism of this invention SCC 1840 was found to resemble the descriptions of Actinomadura fulva and was therefore compared to A. fulva Ferm-P3683 disclosed by A. Tamura and A. Tanaka (Dainippon) in Japanese Patent Publication No. 18035, published April 25, 1984, based on Japanese Kokai 78-28,101, published March 16, 1978 as well as A fulva subsp. uruguayensis SCC 1778, ATCC 53713 , discussed later.

All three cultures are similar in morphology and growth characteristics on various media. All form a substrate mycelium which varies from off-white through yellow to yellow-brown and brown. Both A. fulva Ferm-P3683 and the microorganism of this invention, SCC 1840, form a white, yellowish white or ivory aerial mycelium. A. fulva subsp. uruguayensis SCC 1778, ATCC 53713 tends to form very sparse aerial mycelia, but when visible it is white. The aerial mycelium of all three organisms produce chains of smooth-walled spores which are arranged in tightly appressed spirals forming distinct pseudosporangia. All three microorganisms form only yellow-brown diffusible pigments.

A. fulva Ferm-P3683 differs from the microorganism of this invention SCC 1840 in that A. fulva Ferm-P3683 fails to produce acid from L-arabinose and β-methyl-d-glucopyranoside, does form allantoinase and grows in the presence of 50 µg/ml of neomycin. The microorganism of this invention, SCC 1840, is therefore considered to represent a subspecies of A. fulva.

The microorganism of this invention, SCC 1840, produces a more abundant aerial mycelium on a wider variety of media than does A. fulva subsp. uruguayensis ATCC 53713. SCC 1840 also produces acid from L-arabinose and hydrolyses urea and hippurate while ATCC 53713 does not. SCC 1840 is considered therefore to be a distinct new subspecies of A. fulva designated A. fulva subsp. indica.

TABLE 1

Macroscopic Appearance of Actinomadura fulva subsp. indica SCC 1840, ATCC 53714 on various descriptive media[a]

| MEDIUM | RESULT |
|---|---|
| Yeast Extract-Malt Extract Agar (ISP 2) | G: good to excellent<br>AM: present; abundant, white to ivory (CHM 2db)<br>SC: numerous<br>DFP: present; pale yellow-brown<br>SMP: yellow-brown to grayish brown (ISCC-NBS 61) |
| Oatmeal Agar (ISP 3) | G: fair<br>AM: present; sparse to moderate, white to off-white<br>SC: numerous<br>DFP: present; pale yellow-brown<br>SMP: off-white to moderate brown (ISCC-NBS 58) |
| Inorganic Salts-Starch Agar (ISP 4) | G: fair<br>AM: present; moderate, white<br>SC: numerous<br>DFP: absent<br>SMP: yellow (CHM 2fb, pastel yellow) to yellow-brown |
| Glycerol-Asparagine Agar (ISP 5) | G: good<br>AM: present; sparse (in tufts), white<br>SC: moderate to numerous<br>DFP: present; pale yellow-brown<br>SMP: yellow-brown to brown |
| Peptone-Yeast Extract-Iron Agar (ISP 6) | G: fair<br>AM: present; sparse, white<br>SC: absent<br>DFP: present; pale yellow-brown<br>SMP: yellow-brown to brown |

TABLE I (continued)

| MEDIUM | | RESULT |
|---|---|---|
| AV Agar | G: | fair |
| | AM: | present; sparse, white to yellow-brown |
| | SC: | numerous |
| | DFP: | present; pale yellow-brown |
| | SMP: | Rust brown (CHM 5pg) |
| Gauze's Mineral Agar I | G: | fair to good |
| | AM: | present; sparse, white |
| | SC: | moderate |
| | DFP: | absent |
| | SMP: | pale yellow-brown to brown |
| ATCC Medium 172 | G: | excellent |
| | AM: | present; sparse to moderate, white to ivory (CHM 2db) |
| | SC: | moderate |
| | DFP: | present; yellow-brown |
| | SMP: | deep brown (CHM 6pl) |
| Czapek-Sucrose Agar | G: | poor |
| | AM: | present; sparse to moderate, white |
| | SC: | numerous |
| | DFP: | absent |
| | SMP: | translucent, off-white |
| Glucose-Yeast Extract Agar | G: | excellent |
| | AM: | present; sparse, white |
| | SC: | sparse to numerous |
| | DFP: | yellow-brown |
| | SMP: | moderate brown (ISCC-NBS 58) to dark brown (ISCC-NBS 59) |

[a] G = growth; AM = aerial mycelium; SC = spore chain;
DFP = diffusible pigment;
SMP = substrate mycelium pigmentation

## FERMENTATION OF THE MICROORGANISM

The antimicrobial complex 530 of this invention is produced when the elaborating microorganism, Actinomadura fulva subsp. indica SCC 1840, ATCC 53714 is grown in an aqueous nutrient medium under submerged aerobic conditions at a temperature of about 27° C to 40° C, preferably at from 27° C to 35° C, and at a pH of from about 6.5 to 8.0 with agitation until substantial antimicrobial activity is imparted to the medium. Temperature studies indicate that the organism grows rapidly at about 30° C. Therefore, the fermentation is preferably conducted employing a single temperature pattern of 30° C for a period of about 24 to about 96 hours preferably about 90 hours. The fermentation is generally conducted from about 3 to 7 days, preferably for about 3 days.

To determine when peak antimicrobial production has been reached, samples of the fermentation broth

were assayed every 24 hours (starting at 48 hrs.) for antimicrobial content by bioassay of the whole broth against Staphylococcus aureus ATCC 209P (pH 8.0), Escherichia coli ATCC 10536 (pH 8.0) and Candida albicans Wisconsin. The growth of the organism (packed cell volume), pH and dissolved oxygen levels are determined either intermittantly or continuously.

As nutrient medium, there is employed any suitable medium containing a source of carbon, for example an assimilable carbohydrate, and a source of nitrogen, for example an assimilable nitrogenous or proteinaceous material and various mineral salts.

The medium employed for the fermentation contained meat peptone SB (an enzymatic hydrolysate of casein) and soluble starch as the major sources of nitrogen and carbon, respectively. Under these conditions, the microorganism, SCC 1840, produced antimicrobial complex 530 containing at least one biologically active component as determined by bioautography against both S. aureus, E. coli and C. abicans of the complex after development of a thin layer chromatography plate in 2:2:1 (v/v/v) chlorofrom: methanol: pH 3.5 acetate buffer.

The foregoing media are exemplary of the nutrients utilized by Actinomadura fulva subsp. indica to produce antimicrobial complex 530. However, it is obvious to those trained in the fermentation science that a wide range of nutrients obtained from a number of suppliers may be substituted for the foregoing, and that generally good growth and antibiotic production can be obtained, such nutrients being the functional equivalent to those set forth herein.

The fermentation is generally conducted by initially sterilizing the fermentation medium prior to the addition of the inoculum.

The pH of the fermentation medium is generally maintained at from 6.5 to 8.0, a pH of from 6.5 to 7.5 being preferred. Prior to sterilization, the pH of the medium is usually adjusted to 6.7 and prior to inoculation, the pH is usually adjusted to 7.0.

The fermentation was initiated by addition of the inoculum to the broth. Generally, inoculum volume is 2.5% of total broth volume. The inoculum is prepared by addition of a sample of the frozen whole broth to an appropriate medium. A particularly preferred medium comprises beef extract, 0.3%; tryptone, 0.5%; cerelose, 0.1% potato starch, 2.4%; yeast extract, 0.5%; and calcium carbonate, 0.2% (all percents by weight). The pH of the inoculum medium is adjusted to 7.5 prior to sterilization. The inoculum stage of the fermentation usually requires from 24 to 120 hours with 2 to 4 days preferred and is generally conducted at about 30 °C with agitation. Agitation and a positive air flow, generally about 3.5 L/min. and a temperature of about 30 °C are employed during tank fermentations.

## ISOLATION AND PURIFICATION OF THE ANTIMICROBIAL COMPLEX 530

The antimicrobial complex 530 of this invention contains a complex mixture of antimicrobials, including as the major component, the compound represented by formula I(a), a macrolactam disaccharide along with several minor components. The antimicrobial complex 530 of this invention is isolated by extraction of the whole fermentation broth with n-butanol. The n-butanol extract is concentrated, dissolved in chloroform:methanol (1:1 v/v) and the so formed mixture is added to ether:hexane (6:4 v/v). The so-formed precipitate is the antimicrobial complex of this invention which exhibits antifungal activity against Candida spp. and antibacterial activity against gram positive and negative bacteria.

## ISOLATION AND PURIFICATION OF THE COMPOUND OF THIS INVENTION (Formula I(a))

The antimicrobial complex 530 of this invention was subjected to preparative High Performance Liquid Chromatography (HPLC) on silica gel using chloroform: methanol (95:5 v/v) as the eluting solvent to produce a mixture of macrolactam monosaccharides and the compound represented by formula I(a) which was further purified by using counter-current chromatography with a mobile phase of chloroform:methanol:water (7:10:8 v/v; lower phase). The compound represented by formula I(a) was recovered in substantially chemically pure form from the lower phase. The compound represented by formula I(a) is an unstable compound and breaks down to form a macrolactam monosaccharide having the formula

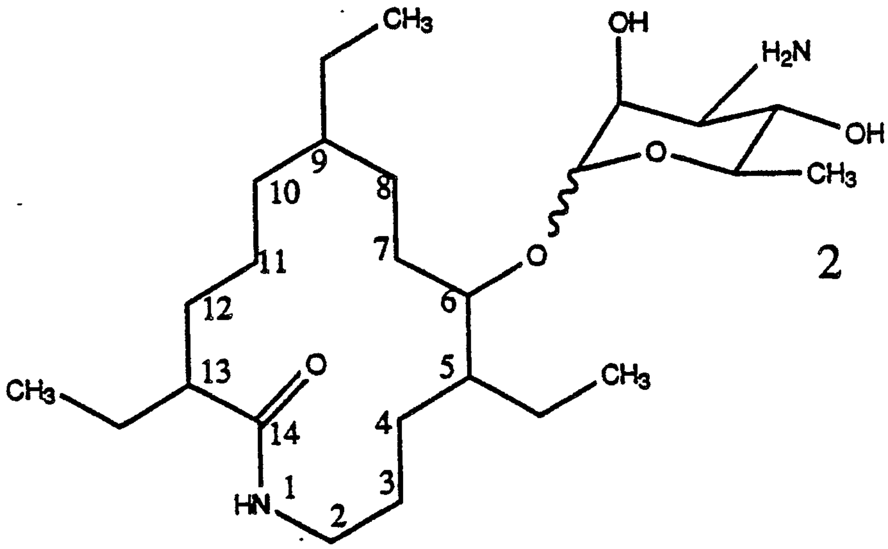

2

The FAB mass spectrum of the compound represented by formula I(a) shows a molecular ion peak at 619 (M+H); high resolution mass measurements reveal the molecular formula of the compound of formula I(a) to be $C_{31}H_{58}N_2O_{10}$. The compound represented by formula I(a) was stabilized by conversion to the hexaacetate derivative. The physiochemical data for the more stable hexaacetate derivative are given in Table II.

## TABLE II

### PHYSIOCHEMICAL DATA FOR HEXAACETATE

| | |
|---|---|
| UV(MeOH) $\lambda$max (nm): | End Absorption |
| IR(KBr) $\nu_{max}$ (cm-1): | 3320, 2930, 1750, 1655, 1540, 1375, 1230, 1050. |
| FAB Mass Spec. : | 871(M+H), 541 |
| $^1H$ NMR($CDCl_3$) : | 5.5(m, 2H), 5.1(t, J=8Hz, 1H) 4.93(t, J=8Hz, 1H), 4.7(m, 4H), 4.15(dd, J=8Hz, 1H), 4.05(d, J=8Hz, 1H), 3.92(dd, J=8,1Hz, 1H), 3.75(dt, J=6,4Hz, 1H), 3.5 (m,4H), 3.1(bt, J=12Hz, 1H), 3.0(bd, J=12Hz, 1H), 2.05(s, 3H), 2.0(s, 6H), 1.96(s, 3H), 1.94(s, 3H), 1.92(s, 3H), 0.9-1.6(m, 26H), 0.7-0.9(m, 9H). |

The $^{13}C$ NMR chemical shifts of the hexaacetate represented by formula I(b) ($R=COCH_3$) are given in Table III.

TABLE III

Carbon-13 NMR Shifts[a,b]

| | Hexacetate formula 2 |
|---|---|
| $-CH_3$ | 9.10 |
| | 12.46 |
| | 12.66 |
| $CH_2$ | 20.79 |
| | 22.49 |
| | 23.12 |
| | 25.06 |
| | 25.27 |
| | 26.77 |
| | 27.25 |
| | 28.23 |
| | 31.78 |
| | 33.73 |
| | 38.66 |

TABLE III (cont'd)

Carbon-13 NMR Shifts[a,b]

| | Hexacetate formula 2 |
|---|---|
| CH | 38.85 |
| | 40.89 |
| | 50.89 |
| | 77.93 |
| O | |
| C | 175.85 |
| SUGAR I | 94.06 |
| | 73.25 |
| | 72.43 |
| | 67.00 |
| | 55.15 |
| | 17.76 |
| $CO\underline{C}H_3$ | 20.79 |
| | 20.79 |
| | 20.79 |
| | 20.79 |
| | 21.20 |
| | 21.66 |
| $\underline{C}OCH_3$ | 169.36 |
| | 169.69 |
| | 169.99 |
| | 170.15 |
| | 170.20 |
| | 170.61 |
| SUGAR II | 89.97 |
| | 74.35 |
| | 72.89 |
| | 71.09 |
| | 68.63 |
| | 61.96 |

[a] all shifts in PPM.
[b] $CDCl_3$ was used as solvent.

Based on the analysis of mass spectral data on the compound represented by formula I(a) and the physicochemical data on the hexaacetate derivative and comparison of the $^{13}C$-NMR data for the hexacetate derivative of the compound of formula I(a), the structure of the compound isolated from antimicrobial complex 530 produced by fermentation of A. fulva subsp. indica ATCC 53714 was shown to be that of formula I(a), a macrolactam disaccharide.

THE BIOLOGICAL ACTIVITY OF THE ANTIMICROBIAL COMPLEX 530, THE COMPOUNDS OF FORMULAS I(a) AND I(b)

The antimicrobial complex 530 of this invention exhibits both antifungal activity and antibacterial activity in vitro against Gram positive and Gram negative microorganisms.

The compound represented by formula I(a), isolated from the antimicrobial complex 530-exhibits in vitro antifungal in a Sabouraud dextrose broth medium against eight species of Candida (geometric mean MIC of 19 mcg/mL) and seven species of dermatophytes (geometric mean MIC of 71 mcg/mL).

The hexacetate derivative, i.e., compound represented by formula I(b) wherein each $R_7$ is $CH_3CO$-, exhibits in vitro antibacterial activity against Gram positive and Gram negative microorganisms, e.g., S. aureus ATCC 209 P, E. coli ATCC 10536, Pseudomonas 720 72401 and Sarcilutia ATCC 9341.

The following examples illustrates preparation of compounds of formulas I(a) and I(b).

## EXAMPLE 1

### Preparation of the Antimicrobial Complex 530 of This Invention

#### A. Inoculum Preparation

##### 1) Initial Stage

Prepare a 250 mL Erlemneyer flask with 70 mL of the following germination medium:

| | |
|---|---|
| Beef Extract | 3 g |
| Tryptone | 5 g |
| Yeast Extract | 5 g |
| Cerelose | 1 g |
| Potato Starch | 24 g |
| Calcium Carbonate | 2 g |
| Tap Water | 1000 mL |
| AF-1* | 1 mL |

*AF-1 is an antifoam agent available from Dow Corning Corp., Midland, MI 48641.

Adjust the pH of the germination broth to 7.5. Sterilize the broth and after cooling, add 3.5 mL of a frozen whole broth sample of the microorganism of this invention from a previously prepared inoculum to each flask broth. Incubate at 30° C with continual agitation at 300 rpm for 48 hours.

##### 2) Second Stage

Transfer 25mL of the first stage germination broth to each of twenty 2-liter Erlenmeyer flasks, each containing 500 mL of the same germination medium and which had been previously pH adjusted and sterilized. Incubate at 30° C with continual agitation at 300 rpm for 48 hours.

#### B. Fermentation

In a 2L fermentor, add 350 ml of the following medium:

| | |
|---|---|
| Cerelose | 25.0 g |
| Soluble Starch | 5.0 g |
| Pro Flo Flour | 10.0 g |
| Marcor Meat Peptone SB | 5.0 g |
| Sodium Nitrate | 1.0 g |
| $ZnSO_4$ $7H_2O$ 5% solution | 1.0 ml |
| $MgCl_2$ $6H_2O$ | 0.5 g |
| $FeSO_4$ $7H_2O$ 1.4% solution | 1.0 ml |
| Tap $H_2O$ | 1000.0 ml |
| Antifoam (AF-1 Dow Corning) | 0.5 ml |

Adjust the pH of the medium to 7.9 and then sterilize the medium. After sterilization, adjust the pH of the medium to 7.0 with a sterile acidic solution. Inoculate the fermentation medium with 25 mL of the second stage inoculum preparation of Step A. Incubate the fermentation mixture at 30° C with a positive air flow and 300 rpm agitation for about 90 hours.

C. Isolation

Extract 5.5L of the fermentation broth two times with equal volumes of n-butanol. Combine the n-butanol solutions, dry over anhydrous sodium sulfate, filter and concentrate by evaporation under reduced pressure. Dissolve the concentrate in 50 mL of chloroform:methanol (1:1 v/v) and then add this solution to 2 liters of ether:hexane (6:4 v/v) until a precipitate results (4.08 g). Filter the precipitate and separate the macrolactam monosaccharides from the compound of formula I(a) by preparative HPLC on a silica gel cartridge using $CHCl_3$; $CH_3OH$ (95:5, v/v) as the developing solvent. The active fractions containing macrolactam disaccharide were combined and the solvent was removed therefrom to provide 765 mg of an active complex. The active complex 530 (57 mg) was placed on an ITO Multicoil Counter Current Chromatographic instrument using the lower phase of the solvent mixture $CHCl_3$:$CH_3OH$:$H_2O$ (7:10:8, v/v/v) as the mobile phase and the upper phase as the stationary phase. The active fractions were identified by TLC and combined. The compound of formula I(a) shows a white lipid-like spot on TLC plates sprayed with water. The solvent was removed from the active fractions to provide 6.2 mg of the compound represented by formula I(a) in substantially chemically pure form. FAB mass spectrum showed a molecular ion peak at 619 (M+H). The molecular formula, $C_{31}H_{58}N_2O_{10}$ was determined by analysis of the high resolution mass spectrum measurements.

## EXAMPLE 2

### Preparation of the Hexaacetate

Acetylate the compound of Example 1 in a stirred mixture of 1:1.5 acetic anhydride-pyridine at 0° C for 3 hours. Stir the reaction mixture overnight at room temperature. Quench the reaction mixture in ice and extract the so-formed mixture with an equal volume of ethyl acetate. Wash the organic extract with dilute HCl and brine. Dry same over anhydrous sodium sulfate to give a crude solid. Chromatograph the crude solid on a silica gel column, eluting with a mixture of ethyl acetate:hexane:methanol (50:50:1.5, v/v/v) to give the pure hexaacetate of formula 2, R = $CH_3CO$-. The physiochemical data for the hexaacetate is provided in Tables II and III.

By substituting the appropriate alkanoic anhydride or alkenoic anhydride for acetic anhydride, the compounds of formula I(b) wherein each $R_7$ is straight or branched chain ($C_1$-$C_{22}$) alkanoyl or straight or branched chain ($C_3$-$C_{22}$) alkenoyl are formed.

### COMPOUND OF FORMULA I HAVING SUBSTITUENTS (C):

(hereinafter Formula I (c))

## THE MICROORGANISM

The microorganism used for the production of antimicrobial complex 517 and the compound represented by formula I(c) is a biologically pure culture of Actinomadura vulgaris subsp. vulgaris sp. nov., subsp. nov. SCC 1776, ATCC 53748.

A viable culture of this microorganism has been deposited in the collection of the American Type Culture Collection (ATCC) in Rockville, Md., where it has been assigned accession number ATCC 53748. The date of deposit is February 23, 1988.

The microorganism was isolated from a sample of soil collected in Borneo. It had been characterized and found to have the microscopic, macroscopic, and whole cell hydrolysis properties of the genus Actinomadura.

## DESCRIPTION OF THE PRODUCING STRAIN: ACTINOMADURA VULGARIS SUBSP. VULGARIS SCC 1776, ATCC 53748

Source material for the following taxonomic evaluations was a frozen preparation of a pure culture of Actinomadura vulgaris subsp. vulgaris SCC 1776, ATCC 53748. Inoculum for the biochemical and physiological tests was prepared according to the procedures of Horan and Brodsky [Horan and Brodsky, Int. J. Syst. Bacteriol., Vol. 32, pp. 195-200 (1982)]. The incubation temperature for the biochemical and physiological tests was 30° C. Readings of the results were made at various times up to 21 days for the plate media. Most of the tubed media were read at various times up to 28 days. The tests for decomposition of urea, allantoin and hippurate, as well as the tests for the reduction of nitrates were read for six weeks.

## MORPHOLOGY

Morphological observations of the producing strain of the microorganism of this invention were made on plates of water agar, AV-agar [Nonomura and Ohara, J. Ferment. Technol., Vol. 47, pp. 463-469 (1966)] or modified inorganic salts-starch agar [Difco inorganic salts-starch agar (ISP-4), 12 g; Difco Bacto agar, 15 g; distilled water, 800 ml; soil extract 200 ml; thiamine HCL, 0.5 mg; riboflavin, 0.5 mg; niacin, 0.5 mg; pyridoxine HCL, 0.5 mg; inositol, 0.5 mg; calcium pantothenate, 0.5 mg; p-aminobenzoic acid, 0.5 mg; biotin, 0.25 mg]. Plates were incubated at 30° C and observed for 4 to 6 weeks.

A. vulgaris subsp. vulgaris is a gram positive filamentous organism that forms a mycelium differentiated into: (1) a substrate mycelium that penetrates the agar and forms a compact surface layer, and (2) an aerial mycelium that originates from the substrate mycelium. The substrate mycelia are well developed and composed of moderately branching, non-fragmenting hyphae approximately 0.4 µm to 0.8 µm in diameter. Spores do not appear to be present. The aerial mycelia are approximately 0.5 µm to 1.0 µm in diameter and bear chains having 6 to 29 spores per chain. The spore chains are straight, hooked, irregularly curved or arranged in spirals of 2 to 5 turns. The spirals are most often loosely coiled or may be tightly appressed. The spores are usually round to ovoid, 0.8 µm to 1.1 µm in diameter. The spore surface is irregularly folded. Motile elements are not formed in either the substrate or aerial mycelia.

## CHEMOTAXONOMY

Purified cell wall preparations of the producing strain of this invention were analyzed by the method of. Becker [Becker et. al., Appl, Microbiol., Vol. 12, pp. 421-423 (1964)] and shown to contain the mesoisomer of 2,6-diaminopimelic acid, alanine, glutamic acid, glucosamine, muramic acid and a trace of mannose. Whole-cell hydroysates were analyzed by the method of Lechevalier [Lechevalier, M.P., J. Lab. Clin. Med., Vol. 71, pp. 934-944 (1968)] and shown to contain glucose, mannose, madurose, ribose, and a trace of rhamnose. The phospholipids present are diphosphatidylglycerol, phosphatidylinositol, phosphatidylinositol-mannosides, phosphatidylethanolamine, a trace of phosphatidylmethylethanolamine and unknown glucosamine-containing phospholipids. Thus, the producing strain of the microorganism of this invention has a type III cell wall with a type B whole-cell sugar pattern and a type P IV phospholipid composition [Lechevalier et. al., Biochem. System. Ecol., Vol. 5, pp. 249-260 (1977)], typical of actinomadurae.

## PHYSIOLOGICAL AND BIOCHEMICAL CHARACTERISTICS

The procedures used to obtain the captional characteristics were those cited by Gordon [Gordon, R.E., J. Gen. Microbiol., Vol. 45, pp. 355-364 (1966)], Luedemann and Brodsky [Luedemann and Brodsky, "Antimicrob. Agents Chemother." pp 47-52 (1965)] and Horan and Brodsky [Horan and Brodsky, Int. J. Syst. Bacteriol., Vol. 32, pp. 195-200 (1982)]. The producing strain, A. vulgaris subsp. vulgaris, SCC 1776, produces acid from adonitol, D-arabinose, L-arabinose, D-fructose, L-fucose, D-galactose, glucose, glycerol, maltose, D-mannitol, D-mannose, α-D-melibiose, α-methyl-D-glucoside, β-methyl-D-glucopyranoside, D-raffinose, α-L-rhamnose, D-ribose, D-sorbitol, sucrose, D-trehalose and D-xylose but not from dulcitol, i-erythritol, or D-melizitose. Adenine, hypoxanthine, L-tyrosine, elastin, xylan, and hippurate are hydrolyzed but guanine, xanthine and chitin are not. Gelatin is both hydrolyzed and liquified. Starch hydrolysis is negative. Urease and allantoinase are not formed. Nitrate is reduced to nitrite. Melanin and hydrogen sulfide are not formed. Growth does not occur at 10° C or at 40° C. Growth is fair at 37° C. The microorganism of this invention, A. vulgaris subsp. vulgaris, SCC 1776, is sensitive to 2% NaCl solution wherein growth is poor. Acetate, formate, lactate, pyruvate and succinate are utilized; benzoate, butyrate and tartrate are not.

A. vulgaris subsp. vulgaris grows in the presence of 50 µg/ml of gentamicin, rifamycin, erythromycin, penicillin G, cephalothin, novobiocin and spectinomycin, and in the presence of 10 mcg/ml gentamicin, sisomicin, streptomycin and neomycin. Growth is poor in the presence of 50 mcg/ml of sisomicin, neomycin, kanamycin, clindamycin and everninomicin.

## DESCRIPTION OF A. VULGARIS SUBSP. VULGARIS ON VARIOUS MEDIA

All plates were incubated at 30° C and observed at intervals up to 28 days. The common names for the colors were choosen after comparison with color chips from the ISCC-NBS centroid color charts, the "Color Harmony Manual", Ed. 4 (Container Corp. America, 1958), or the "Methuen Handbook of Color" (Eyre Methuen, London, 1981). On all media tested, the substrate mycelium of A. vulgaris subsp. vulgaris is pale yellow to yellow-brown. The aerial mycelium is white to ivory. The B-vitamins are not essential for growth but seem to exhance production of aerial mycelia and spores. Yellow-brown soluble pigments are produced. Characteristics are presented in Table IV.

Based on the yellow-brown pigmentation of the substrate mycelia, spore morphology and physilogical characteristics A. vulgaris subsp. vulgaris SCC 1776 (ATCC 53748) appears to be most closely related to A. vulgaris subsp. lanata ATCC 53715 (descriped later) and A. spiralis IFO 14097.

A. vulgaris subsp. vulgaris ATCC 53748 and A. spiralis IFO 14097 differ significantly. In direct comparisons, A. spiralis was found to form shorter spore chains (10 to 16 spores per chain); to hydrolyze starch; to form urease and allantoinase; to grow in the presence of 50 mcg/ml of everninomicin but not to grow in the presence of gentamicin, sisomicin and neomycin at 10 mcg/ml; not to hydrolyze xylan or to produce acid from D-sorbitol or sucrose and not to exhibit antibiotic activity.

A. vulgaris subsp. lanata SCC 1777 is similar to A. vulgaris subsp. vulgaris ATCC 53748 in substrate mycelial pigmentation, morphology of the spore-bearing hyphae, biochemical and physiological characteristics and antibiotic production. They differ in growth temperature range, resistance to novobiocin and acid production from α-methyl-D-glucoside and D-raffinose. In addition, the macroscopic appearance of the aerial mycelia of A. vulgaris subsp. lanata SCC 1777 (ATCC 53715) is consistently wooly in texture when compared to A. vulgaris subsp. vulgaris ATCC 53748. Based on these differences, ATCC 53748 is

considered to be a distinct new species designated A. vulgaris sp. nov. Shearer, Brodsky and Horan.

In accordance with the Rules of Nomenclature of Bacteria [Lapage, Sneath, Lessel, Skerman, Seeliger and Clark, Ed. 1975 Int. Code of Nomenclature of Bacteria, 1976 rev. Am. Soc. Microbiol., Washington, D.C.] this is both the type strain and the type subspecies, A. vulgaris subsp. vulgaris.

The species and subspecies names selected (vulgaris, L. adj. common, ordinary) refers to the frequency with which this microorganism can be isolated from soil.

TABLE IV

Macroscopic Appearance of Actinomadura vulgaris subsp. vulgaris SCC 1776, ATCC 53748 on various descriptive media[a]

| MEDIUM | | RESULT |
|---|---|---|
| Yeast Extract-Malt Extract Agar (ISP 2) | G: | good to excellent |
| | AM: | present; sparse to moderate; white to ivory (CHM 2db) |
| | SC: | numerous |
| | DFP: | present; light yellow-brown |
| | SMP: | yellow-brown (CHM-2fb, buff, straw |
| Oatmeal Agar (ISP 3) | G: | good |
| | AM: | present; sparse, white |
| | SC: | numerous |
| | DFP: | absent |
| | SMP: | yellow-brown |
| Inorganic Salts-Starch Agar (ISP 4) | G: | fair to good |
| | AM: | present; sparse to moderate, white |
| | SC: | numerous |
| | DFP: | absent |
| | SMP: | yellow-brown |
| Glycerol-Asparagine Agar (ISP 5) | G: | fair to good |
| | AM: | present; sparse to moderate, white |
| | SC: | sterile to moderate |
| | DFP: | absent |
| | SMP: | yellow (CHM-2ic, lt. gold) to yellow-brown |
| Peptone-Yeast Extract-Iron Agar (ISP 6) | G: | fair |
| | AM: | absent |
| | SC: | absent |
| | DFP: | absent |
| | SMP: | yellow-brown |

TABLE IV (continued)

| MEDIUM | | RESULT |
|---|---|---|
| AV Agar | G: | fair |
| | AM: | present abundant, ivory (CHM-2db) |
| | SC: | numerous |
| | DFP: | absent |
| | SMP: | yellow-brown |
| Gauze's Mineral Agar I | G: | good |
| | AM: | present; sparse, white |
| | SC: | sterile |
| | DFP: | absent |
| | SMP: | yellow to yellow-brown |
| ATCC Medium 172 | G: | excellent |
| | AM: | absent |
| | SC: | absent |
| | DFP: | present; yellow-brown |
| | SMP: | yellow-brown |
| Czapek-Sucrose Agar | G: | fair to good |
| | AM: | present; sparse, white |
| | SC: | sparse |
| | DFP: | absent |
| | SMP: | off-white to pale yellow-brown |
| Glucose-Yeast Extract Agar | G: | excellent |
| | AM: | absent |
| | SC: | absent |
| | DFP: | absent |
| | SMP: | yellow-brown |

a G = growth; AM = aerial mycelium; SC = spore chain;
DFP = diffusible pigment;
SMP = substrate mycelium pigmentation

## FERMENTATION OF THE MICROORGANISM

The antimicrobial complex 517 of this invention is produced when the elaborating microorganism, Actinomadura vulgaris subsp. vulgaris SCC 1776, ATCC 53748 is grown in an aqueous nutrient medium under submerged aerobic conditions at a temperature of about 27° C to 40° C, preferably at from 27° C to 35° C, and at a pH of from about 6.5 to 8.0 with agitation until substantial antimicrobial activity is imparted to the medium. Temperature studies indicate that the organism grows rapidly at about 30° C. Therefore, the fermentation is preferably conducted employing a single temperature pattern of about 30° C for a period of about 24 to about 96 hours preferably about 90 hours. The fermentation is generally conducted from about 3 to 7 days, preferably for about 3 days.

To determine when peak antimicrobial production has been reached, samples of the fermentation broth were assayed every 24 hours (starting at 48 hrs.) for antimicrobial content by bioassay of the whole broth against Staphylococcus aureus ATCC 209P (pH 8.0), Escherichia coli ATCC 10536 (pH 8.0) and Candida albicans Wisconsin. The growth of the organism (packed cell volume), pH and dissolved oxygen levels are determined either intermittantly or continuously.

As nutrient medium, there is employed any suitable medium containing a source of carbon, for example

an assimilable carbohydrate, and a source of nitrogen, for example an assimilable nitrogenous or proteinaceous material and various mineral salts.

The medium employed for the fermentation contained potatoe dextrin, cottonseed flour and crushed pea, molasses and maltose as the major sources of nitrogen and carbon, respectively. Under these conditions, the microorganism, SCC 1776, produced antimicrobial complex 517 containing at least seven biologically active components as determined by bioautography against both S. aureus, E. coli and C. albicans of the complex after development of a thin layer chromatography plate in 8:2 (v/v/v) chloroform: methanol.

The foregoing media are exemplary of the nutrients utilized by Actinomadura vulgaris subsp. vulgaris to produce antimicrobial complex 517. However, it is obvious to those trained in fermentation science that a wide range of nutrients obtained from a number of suppliers may be substituted for the foregoing, and that generally good growth and antibiotic production can be obtained,such nutrients being the functional equivalent to those set forth herein.

The fermentation is generally conducted by initially sterilizing the fermentation medium prior to the addition of the inoculum.

The pH of the fermentation medium is generally maintained at from 6.5 to 8.0, a pH of from 6.5 to 7.5 being preferred. Prior to sterilization, the pH of the medium is usually adjusted to 7.2.

The fermentation was initiated by addition of the inoculum to the broth. Generally, inoculum volume is 5.0% of total broth volume. The inoculum is prepared by addition of a sample of the frozen whole broth to an appropriate medium. A particularly preferred medium comprises beef extract, 0.3%; tryptone, 0.5%; cerelose, 0.1%; potato starch, 2.4%; yeast extract, 0.5%; and calcium carbonate, 0.2% (all percents by weight). The inoculum stage of the fermentation usually requires from 24 to 120 hours with 2 to 4 days preferred and is generally conducted at about 30°C with agitation. Agitation and a positive air flow, generally about 4.5 L/min. and a temperature of about 30°C are employed during the fermentation. A particularly preferred fermentation medium comprises potatoe dextrin 2.0%; cottonseed flour 0.75% crushed pea 0.25%; molasses 0.5%; maltose 0.5%; and calcium carbonate 0.4%. The pH of the solution is adjusted to 7.2 prior to the addition of calcium carbonate. An antifoam agent such as SAG (Union Carbide Corp., 50% solution) is added, if necessary, to the fermentors to control foam.

## ISOLATION AND PURIFICATION OF THE ANTIMICROBIAL COMPLEX 517

The antimicrobial complex 517 of this invention contains a complex mixture of antimicrobials, including as the major component, the compound represented by formula I(c), a macrolactam monosaccharide along with a known macrolactam monosaccharide represented by formula 3 and five minor components. The antimicrobial complex 517 of this invention is isolated by extraction of the whole fermentation broth with n-butanol. The n-butanol extract is washed, dried, concentrated, and dissolved in methanol; the so-formed mixture is added to ether:hexane (6:4 v/v). The so-formed precipitate is the antimicrobial complex 517 of this invention which exhibits antifungal activity against Candida spp. and antibacterial activity against gram positive and negative bacteria.

## ISOLATION AND PURIFICATION OF THE COMPOUND OF THIS INVENTION (Formula I(c))

The compound of formula I(c) was obtained as the major antimicrobial component of the antimicrobial complex 517 by preparative High Performance Liquid Chromatography (HPLC) of antimicrobial complex 517 on a silica gel column using as the eluate a mixture of chloroform:methanol:triethylamine (95:5:0.5, v/v/v).

The compound represented by formula I(c) is a white solid, basic in nature and gives a positive color reaction with ninhydrin. The compound is fairly soluble in methanol, dimethyl sulfoxide, sparingly soluble in ethyl acetate and insoluble in water and also shows a white lipid like spot on TLC plate when sprayed with water.

The physicochemical data for the compound represented by formula I(c) are shown in Table V.

TABLE V

| PHYSIOCHEMICAL DATA FOR COMPOUND OF FORMULA I(c) | |
|---|---|
| Opt. Rot : | $[\alpha]_D^{26}$ = -5.8 ($CH_3OH$, c 0.5) |
| UV λmax : | End Absorption |
| IR(KBr) : | 3420, 3300, 2920, 1640, 1555, 1385, 1050 $cm^{-1}$ |
| FAB MS : | 457(M+H), 312, 294, 163, 146 |
| PMR($CD_3OD$ + $CDCl_3$) δ : | 0.85(t, 3 -$CH_3$), 1.25(d, -$CH_3$), 1.0-1.7($CH_2$ & CH), 2.05(m, 1H), 3.03(dd, CH), 3.3(m, CH), 3.4-3.7(several CH), 4.9(d, 1H). |
| Molecular Formula: | $C_{25}H_{48}N_2O_5$ |

The compound represented by formula 3 hereinbelow was isolated from antimicrobial complex 517 as a white crystalline solid:

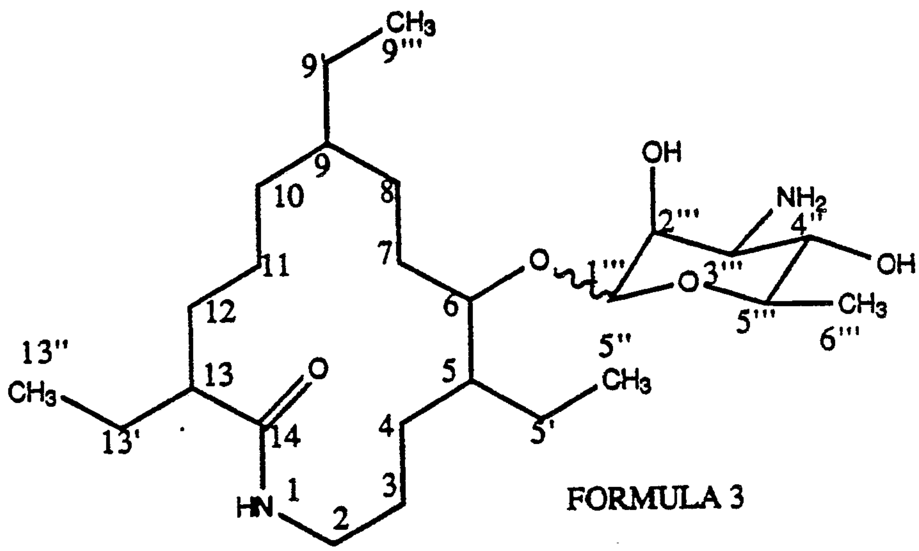

FORMULA 3

We determined that (1) the compound of formula 3 is basic in nature and shows a positive color reaction wth ninhydrin, is fairly soluble in methanol and dimethyl sulfoxide, sparingly soluble in ethyl acetate and chloroform and insoluble in water, and (2) the compound of formula 3 like all the components from antimicrobial complex 517 is lipophillic in nature and shows a white lipid like spot on TLC plates which are sprayed with water. Physiochemical data for compound of formula 3 are given in Table VI.

TABLE VI

| PHYSIOCHEMICAL DATA FOR COMPOUND OF FORMULA 3 | |
|---|---|
| Opt. Rot : | $[\alpha]_D^{26}$ = +9.7 (c 0.5, MeOH) |
| UV λmax : | End Absorption |
| IR (KBr) : | 3420, 3300, 2930, 1640, 1550, 1455, 1045 $cm^{-1}$. |
| FAB MS : | 457.3674(M+H) |
| Mol. Formula : | $C_{25}H_{48}N_2O_5$ |
| PMR($CD_3OD$+$CDCl_3$) δ : | 0.82(t, $CH_3$), 0.89(t, 2-$CH_3$) 1.25(d, $CH_3$), 1.0-1.8($CH_2$ & CH) 2.05(m, 1H), 2.90(dd, 1H), 3.00(bd, 1H), 3.25(t, 1H) 3.65(m, 1H), 3.75(bd, 1H), 4.80(d, 1H). |

The Carbon-13 NMR data for the compounds of formulas I(c) and 3 are given in Table VII

TABLE VII

| COMPARISON OF CMR DATA OF COMPOUNDS OF FORMULA I(c) AND 3 | | |
|---|---|---|
| Carbon | Compound of Formula I(c) | Compound of Formula 3 |
| $CH_3$ | 8.95, 12.26, 12.60 16.68 | 8.95, 12.28, 12.56 17.67 |
| $CH_2$ | 21.57, 21.82, 22.79 25.51, 25.57, 26.94 27.69, 28.08, 32.57, 33.93, 39.20 | 21.47, 21.74, 22.69, 25.55, 25.55, 26.90, 27.63, 28.07, 32.55, 33.90, 39.21 |
| CH | 38.95, 41.24, 49.40, 50.86, 62.89, 67.47, 69.33, 77.59, 97.50 | 38.95, 41.22, 50.66, 53.84, 69.82, 71.00 72.91, 76.95, 97.49 |
| CO | 178.23 | 178.17 |

The compound of formula I(c) is levorotatory with a specific rotation of $[\alpha]_D^{26}$ = -5.8 and has no UV absorption. FAB mass spectrum shows $(M+H)^+$ peak at 457 and thus, the molecular weight of the compound of formula I(c) is 456. The characteristic peaks in IR at 1640 and 1550 $cm^{-1}$ indicate the presence of an amide. The physicochemical data of Tables V, VI and VII and derivatization and degradation studies on the compounds of formula I(c) and 3 are consistent with a macrolactam monosaccharide having the structure represented by the formula I(c).

## THE BIOLOGICAL ACTIVITY OF THE ANTIMICROBIAL COMPLEX 517, AND THE COMPOUND OF FORMULA I-(c)

The antimicrobial complex 517 of this invention exhibits both antifungal activity and antibacterial activity in vitro against Gram positive and Gram negative microorganisms.

The compound represented by formula I(c), isolated from the antimicrobial complex 517, exhibits in vitro antifungal in a Sabouraud dextrose broth medium against seven species of Candida (geometric mean MIC of 2.0 mcg/mL) and six species of dermatophytes (geometric mean. MIC of 101.6 mcg/mL) and antifungal activity against seven species of. Candida (geometric mean MIC of 6.56 mcg/mL) in Eagles Minimum Essential Medium.

The following example illustrates preparation of the compound of Formula I(c).

## EXAMPLE 3

### Preparation of the Antimicrobial Complex 517 of This Invention

The compound of formula I(c) was produced as the major component of antimicrobial complex 517 by natural strain selection of the A. vulgaris subsp. vulgaris, SCC 1776, ATCC 53748.

### A. Inoculum Preparation

#### 1) Initial Stage

Prepare a 250 mL Erlemneyer flask with 50 mL of the following germination medium:

| | |
|---|---|
| Beef Extract | 3 g |
| Tryptone | 5 g |
| Yeast Extract | 5 g |
| Cerelose | 1 g |
| Potato Starch | 24 g |
| Calcium Carbonate | 2 g |
| Tap Water | 1000 mL |
| AF-1* | 1 mL |

*AF-1 is an antifoam agent available from Dow Corning Corp., Midland, MI 48641.

Sterilize the broth and after cooling, add 2.5 mL of a frozen whole broth sample of the microorganism of this invention from a previously prepared inoculum to each flask broth. Incubate at 30° C with continual agitation at 300 rpm for 48 hours.

2) Second Stage

Transfer 25mL of the first stage germination broth to each of twenty 2-liter Erlenmeyer flasks, each containing 350 mL of the same germination medium and which had been previously pH adjusted and sterilized. Incubate at 30° C with continual agitation at 300 rpm for 48 hours.

B. Fermentation

In a 150L fermentor, add 100L of the following medium:

| | g/l |
|---|---|
| Potatoe Dextrin | 20.0 |
| Cottonseed Flour | 7.5 |
| Crushed Pea | 2.5 |
| Molasses | 5.0 |
| Maltose | 5.0 |
| $CaCO_3$ | 2.0 |
| Tap Water | 1000.0 mL |
| Antifoam (SAG 471 Union Carbide 50% Solution) | 1.0 mL |

Adjust the pH of the medium to 7.2 and then sterilize the medium. Inoculate the fermentation medium with 700 mL of the second stage inoculum preparation of Step A. Incubate the fermentation mixture at 30° C with 18 cubic feet per minute of air flow and 400 rpm agitation for about 72 hours.

C. Isolation

Extract 100L of the whole fermentation broth produced in accordance with the fermentation procedure hereinabove with two 50L volumes of n-butanol. Concentrate the combined n-butanol extracts to 2L and wash the 2L concentrate three times with 2L of water. Concentrate the washed n-butanol extracts to dryness and dissolve the concentrate in 200 mL of chloroform: methanol (1:1, v/v). Add to the so-formed solution, 5 L of diethyether:hexane (5:2, v/v) to produce 9.5g of crude antimicrobial complex 517 comtaining the compound of formula I(c) as the major component.

Purify the crude antimicrobial complex 517 on preparative HPLC (Water Associates, Preparative 500) using silica gel cartridges and as eluent chloroform:methanol:triethylamine (95:5:0.5, v/v/v) to produce 1.18g of the compound of formula I(c) as a white solid m.p.: 216-220° C (dec). The white solid is substantially

chemically pure, basic in nature, gives a positive color reaction with ninhydrin, is fairly soluble in methanol, dimethylsulfoxide, is sparingly soluble in ethyl acetate and insoluble in water. The physicochemical data for the compound of formula I(c) are summarized in Tables V and VII.

## COMPOUNDS OF FORMULA I HAVING SUBSTITUENTS (d):

(hereinafter Compounds of Formula I(d))

## THE MICROORGANISM

The microorganism used for the production of antimicrobial complex 510 and the compound represented by formula I(d) is a biologically pure culture of Actinomadura fulva subsp. uruguayensis subsp. nov. Shearer, Brodsky and Horan SCC 1778, ATCC 53713.

A viable culture of this microorganism has been deposited in the collection of the American Type Culture Collection (ATCC) in Rockville, Md., where it has been assigned accession number ATCC 53713. The deposit date is January 25, 1988.

The microorganism was isolated from a sample of soil collected in Uruguay. It had been characterized and found to have the microscopic, macroscopic, and whole cell hydrolysis properties of the genus Actinomadura.

## DESCRIPTION OF THE PRODUCING STRAIN: ACTINOMADURA FULVA SUBSP. URUGUAYENSIS SCC 1778, ATCC 53713

Source material for the following taxonomic evaluations was a frozen preparation of a pure culture of Actinomadura fulva subsp. uruguayensis SCC 1778, ATCC 53713. Inoculum for the biochemical and physiological tests was prepared according to the procedures of Horan and Brodsky [Horan and Brodsky, Int. J. Syst. Bacteriol., Vol. 32, pp. 195-200 (1982)]. The incubation temperature for the biochemical and physiological tests was 30 °C. Readings of the results were made at various times up to 21 days for the plate media. Most of the tubed media were read at various times up to 28 days. The tests for decomposition of urea, allantoin and hippurate, as well as the tests for the reduction of nitrates were read for six weeks.

## MORPHOLOGY

Morphological observations of the producing strain of the microorganism of this invention were made on plates of water agar, AV-agar [Nonomura and Ohara, J. Ferment. Technol., Vol. 47, pp. 463-469 (1966)] or modified inorganic salts-starch agar [Difco inorganic salts-starch agar (ISP-4), 12 g; Difco Bacto agar, 15 g; distilled water, 800 ml; soil extract 200 ml; thiamine HCL, 0.5 mg; riboflavin, 0.5 mg; niacin, 0.5 mg; pyridoxine HCL, 0.5 mg; inositol, 0.5 mg; calcium pantothenate, 0.5 mg; p-aminobenzoic acid, 0.5 mg; biotin, 0.25 mg]. Plates were incubated at 30 °C and observed for 4 to 6 weeks.

A. fulva subsp. uruguayensis is a gram positive, filamentous organism that forms a mycelium differentiated into: (1) a substrate mycelium that penetrates the agar and forms a compact surface layer, and (2) an aerial mycelium that originates from the substrate mycelium.

The substrate mycelium is well developed with moderately branching, non-fragmenting hyphae which are approximately 0.4 µm to 0.8 µm in diameter. No spores are observed on the substrate hyphae.

The aerial hyphae, which are approximately 0.6 µm to 1.o µm in diameter, bear chains of spores. These spore chains contain approximately 6 to 23 spores per chain. The smooth walled spores are round to ovoid and approximately 1.1 to 1.5 µm in diameter. The spore chains are arranged in tightly appressed

spirals, forming pseudosporangia approximately 1.5 to 5.5 µm in diameter. No motile elements are observed in either the substrate or aerial mycelium.

## CHEMOTAXONOMY

Purified cell wall preparations of the producing strain of this invention were analyzed by the method of Becker [Becker et. al,, Appl, Microbiol., Vol. 12, pp. 421-423 (1964)] and shown to contain the mesoisomer of 2,6-diaminopimelic acid, alanine, glutamic acid, glucosamine, muramic acid and traces of mannose. Whole-cell hydroysates were analyzed by the method of Lechevalier [Lechevalier, M.P., J. Lab. Clin. Med., Vol. 71, pp. 934-944 (1968)] and shown to contain glucose, mannose, madurose, ribose, a trace of galactose and a trace of rhamnose. The phospholipids present are diphosphatidylglycerol, phosphatidylinositol, phosphatidylethanolamine and unknown glucosamine-containing phospholipids. Thus, the producing strain of the microorganism of this invention, SCC 1778, has a type III cell wall with a type B whole-cell sugar pattern and a type P IV phospholipid composition [Lechevalier et. al., Biochem. System. Ecol., Vol. 5, pp. 249-260 (1977)], typical of actinomadurae.

## PHYSIOLOGICAL AND BIOCHEMICAL CHARACTERISTICS

The procedures used were those cited by Gordon [Gordon, R.E., J. Gen. Microbiol., Vol. 45, pp. 355-364 (1966)], Luedemann and Brodsky [Luedemann and Brodsky, "Antimicrob. Agents Chemother." pp 47-52 (1965)] and Horan and Brodsky [Horan and Brodsky, Int. J. Syst. Bacteriol., Vol. 32, pp. 195-200 (1982)]. The producing strain, A. fulva subsp. uruguayensis, SCC 1778, produces acid from adonitol, D-arabinose, D-fructose, L-fucose, D-galactose, glucose, glycerol, i-inositol, maltose, D-mannitol, D-mannose, α-D-melibiose, α-methyl-D-glucoside, β-methyl-D-glucopyranoside, α-L-rhamnose, D-ribose, sucrose, D-treholose and D-xylose but not from L-arabinose dulcitol, i-erythritol, D-melizitose, or D-sorbitol. Adenine, hypoxanthine, L-tyrosine, elastin and casein are hydrolyzed but guanine, xanthine, xylan hippurate and chitin are not. Gelatin is both hydrolyzed and liquified. Starch hydrolysis is negative. Urease and allantoinase are not formed. Nitrate is reduced to nitrite. Melanin and hydrogen sulfide are not formed. Growth does not occur at 10 °C or at 45 °C. Growth is fair at 40 °C. The microorganism of this invention, SCC 1778, grows in the presence of 3% NaCl but grows poorly to fairly at 4% NaCl solution. Acetate, butyrate, lactate, pyruvate and succinate are utilized; benzoate, formate, oxalate and tartrate are not.

A. fulva subsp. uruguayensis grows in the presence of 50 µg/ml of streptomycin, kanamycin, novobiocin, rifamycin, erythromycin, penicillin G, cephalothin, tetracycline, cycloserine and spectinomycin and in the presence of 10 mcg/ml of neomycin. Growth is poor in the presence of 50 mcg/ml of sisomicin, gentamicin and clindamycin.

## DESCRIPTION OF A. FULVA SUBSP. URUGUAYENSIS ON VARIOUS MEDIA

All plates were incubated at 30 °C and observed at intervals up to 28 days. The common names for the colors were choosen after comparison with color chips from the ISCC-NBS centroid color charts, the "Color Harmony Manual" Ed. 4 (Container Corp. America, 1958), or the "Methuen Handbook of Color" (Eyre Methuen, London, 1981). On all media tested, the substrate mycelium of A. fulva subsp. uruguayensis is off-white, yellow-brown or brown. Few aerial mycelia are produced by SCC 1778 but when visible, the aerial mycelia are white to ivory to yellowish-gray. Yellow-brown diffusible pigments are produced. Characteristics are presented in Table 1.

On the basis of its morphological and chemotaxonomic characteristics, the producing strain of of this invention, SCC 1778, was placed in the genus Actinomadura. The description of the microorganism of this invention, SCC 1778, was compared to the descriptions of Actinomadura species found on the approved lists of bacterial names or in the patent literature. The microorganism of this invention, SCC 1778, was found to resemble the descriptions of Actinomadura fulva and was therefore compared to A. fulva Ferm-P3683 disclosed by A. Tamura and A. Tanaka (Dainippon) in Japanese Patent Publication No. 18035, published April 25, 1984, based on Japanese Kokai 78-28,101, published March 16, 1978 as well as A. fulva

subsp. indica SCC 1840, ATCC 53714, discussed previously.

All three cultures are similar in morphology and growth characteristics on various media. All form a substrate mycelium which varies from off-white through yellow to yellow-brown and brown. The Japanese Patent discloses that A. fulva Ferm P-3683 produces pink aerial mycelium. In our side-by-side comparisons, both A. fulva Ferm-P3683 and A. fulva subsp. indica SCC 1840, form a white, yellowish white or ivory aerial mycelium. A. fulva subsp. uruguayensis SCC 1778, ATCC 53713 tends to form very sparse aerial mycelia, but when visible, the aerial mycelia are white. The aerial mycelium of all three organisms produce chains of smooth-walled spores which are arranged in tightly appressed spirals forming distinct pseudosporangia. All three microorganisms form only yellow-brown diffusible pigments.

A. fulva Ferm-P3683 differs from the microorganism of this invention, SCC 1778, in that A. fulva Ferm-P3683 fails to produce acid from i-inositol and β-methyl-d-glucopyranoside, does form allantoinase and grows in the presence of 50 µg/ml of neomycin. The microorganism of this invention, SCC 1778, is therefore considered to represent a subspecies of A. fulva.

The microorganism of this invention, SCC 1778, ATCC 53713 produces a less abundant aerial mycelium on a wider variety of media than does A. fulva subsp. indica ATCC 53715. A. fulva subsp. indica also produces acid from L-arabinose and hydrolyses urea and hippurate while the microorganism of this invention, SCC 1778, ATCC 53713 does not. SCC 1778 is considered therefore to be a distinct new subspecies of A. fulva designated A. fulva subsp. uruguayensis SCC 1778, ATCC 53713.

TABLE VIII

Macroscopic Appearance of Actinomadura fulva subsp. uruguayensis SCC 1778, ATCC 53713 on various descriptive media[a]

| MEDIUM | RESULT |
|---|---|
| Yeast Extract-Malt Extract Agar (ISP 2) | G: good<br>AM: none to sparse, white<br>SC: moderate to numerous<br>DFP: absent<br>SMP: brown |
| Oatmeal Agar (ISP 3) | G: fair<br>AM: not visible<br>SC: sparse to moderate<br>DFP: pale yellow-brown<br>SMP: translucent to yellow-brown (CHM-2fb, bamboo, buffer) |
| Inorganic Salts-Starch Agar (ISP 4) | G: fair<br>AM: not visible<br>SC: sparse to moderate<br>DFP: absent<br>SMP: translucent to yellow-brown |
| Glycerol-Asparagine Agar (ISP 5) | G: fair to good<br>AM: not visible<br>SC: sparse to moderate<br>DFP: absent<br>SMP: translucent to yellow-brown |
| Peptone-Yeast Extract-Iron Agar (ISP 6) | G: fair to good<br>AM: not visible<br>SC: absent<br>DFP: pale yellow-brown<br>SMP: yellow-brown to brown (CHM 4ng, lt. brown, maple) |
| AV Agar | G: fair<br>AM: not visible<br>SC: absent<br>DFP: absent<br>SMP: yellow-brown to brown |
| Gauze's Mineral Agar I | G: fair<br>AM: not visible<br>SC: absent<br>DFP: absent<br>SMP: ivory (CHM 2db) to pale yellow-brown |

| | | |
|---|---|---|
| ATCC Medium 172 | G: | excellent |
| | AM: | not visible |
| | SC: | absent |
| | DFP: | yellow-brown |
| | SMP: | brown (CHM 4ng, Lt. brown, maple) |
| Czapek-Sucrose Agar | G: | poor |
| | AM: | not visible |
| | SC: | sparse to moderate |
| | DFP: | absent |
| | SMP: | translucent, off-white to pale yellow-brown |
| Glucose-Yeast Extract Agar | G: | excellent |
| | AM: | not visible |
| | SC: | absent |
| | DFP: | pale yellow-brown |
| | SMP: | yellow-brown to brown (CHM 4pr, oak brown) |

[a] G = growth; AM = aerial mycelium; SC = spore chain; DFP = diffusible pigment; SMP = substrate mycelium pigmentation

## FERMENTATION OF THE MICROORGANISM

The antimicrobial complex 510 of this invention is produced when the elaborating microorganism, Actinomadura fulva subsp. uruguayensis SCC 1778, ATCC 53713 is growth in an aqueous nutrient medium under submerged aerobic conditions at a temperature of about 27° C to 40° C, preferably at from 27° C to 35° C, and at a pH of from about 6.5 to 8.0 with agitation until substantial antimicrobial activity is imparted to the medium. Temperature studies indicate that the organism grows rapidly at about 30° C. Therefore, the fermentation is preferably conducted employing a single temperature pattern of 30° C for a period of about 24 to about 96 hours preferably about 90 hours. The fermentation is generally conducted from about 3 to 7 days, preferably for about 4 days (90 hrs.).

To determine when peak antimicrobial production has been reached, samples of the fermentation broth were assayed every 24 hours (starting at 48 hrs.) for antimicrobial content by bioassay of the whole broth against Staphylococcus aureus ATCC 209P (pH 8.0), Escherichia coli ATCC 10536 (pH 8.0) and Candida albicans Wisconsin. The growth of the organism (packed cell volume), pH and dissolved oxygen levels are determined either intermittantly or continuously.

As nutrient medium, there is employed any suitable medium containing a source of carbon, for example an assimilable carbohydrate, and a source of nitrogen, for example. an assimilable nitrogenous or proteinaceous material and various mineral salts.

The medium employed for the fermentation contained beet extract (an enzymatic hydrolysate of casein) and insoluble starch as the major sources of nitrogen and carbon, respectively. Under these conditions, the microorganism, SCC 1778, produced antimicrobial complex 510 containing at least one biologically active component as determined by bioautography against both S. aureus, E. coli and C. abicans of the complex after development of a thin layer chromatography plate in 2:2:1 (v/v/v) chloroform: methanol: pH 3.5 acetate buffer.

The foregoing media are exemplary of the nutrients utilized by Actinomadura fulva subsp. uruguayensis to produce antimicrobial complex 510. However, it is obvious to those trained in the fermentation science that a wide range of nutrients obtained from a number of suppliers may be substituted for the foregoing, and that generally good growth and antibiotic production can be obtained, such nutrients being the functional equivalent to those set forth herein.

The fermentation is generally conducted by initially sterilizing the fermentation medium prior to the

addition of the inoculum.

The pH of the fermentation medium is generally maintained at from 6.5 to 8.0, a pH of from 6.5 to 7.5 being preferred. Prior to sterilization, the pH of the medium is usually adjusted to 7.0.

The fermentation was initiated by addition of the inoculum to the broth. Generally, inoculum volume is 3.0% of total broth volume. The inoculum is prepared by addition of a sample of the frozen whole broth to an appropriate medium. A particularly preferred inoculum preparation medium comprises beef extract, 0.3%; tryptone, 0.5%; cerelose, o.1% potato starch, 2.4%; yeast extract, 0.5%; and calcium carbonate, 0.2% (all percents by weight). The inoculum stage of the fermentation usually requires from 24 to 120 hours with 2 to 4 days preferred and is generally conducted at about 30° C with agitation. Agitation and a positive air flow, generally about 3.5 L/min. a 90 hour incubation period and a temperature of about 30° C are employed during the fermentations medium comprises cottonseed flower, 1.0%; peptone 0.1%; cerelose, 5.0%; cornsteep liquor, 0.5 vol. %; arabinose, 0.5%, $MgCl_2 \cdot 6H_2O$, 0.05% and 0.1 vol. % of each of: (1) a 5 wt % solution of $ZnSO_4 \cdot 7H_2O$; and (2) a 1.4 wt % solution of $FeSO_4 \cdot 7H_2O$. The pH of the solution is adjusted to 7 prior to the addition of mineral salts. An antifoam agent such as Antifoam AF-1 (DOW Corning) is added, if necessary, to the fermentors to control foam.

## ISOLATION AND PURIFICATION OF THE ANTIMICROBIAL COMPLEX 510

The antimicrobial complex 510 of this invention contains a complex mixture of antimicrobials, including as the major components the three compounds represented by formula I(d), macrolactam monosaccharides along with a fourth macrolactam monosaccharide of formula 4 as well as several minor components. The antimicrobial complex 510 of this invention is isolated by extraction of the whole fermentation broth at a pH of 9.5 with ethyl acetate. The ethyl acetate extracts were shaken with water at pH of 2. The aqueous layer was extracted with n-butanol. The n-butanol extract is washed, dried, concentrated, dissolved in methanol; the so formed mixture is added to an ether:hexane mixture. The so-formed precipitate is the antimicrobial complex 510 of this invention which exhibits antifungal activity against fungi such as *Candida* sp. and antibacterial activity against Gram positive and gram negative bacteria.

## ISOLATION AND PURIFICATION OF THE COMPOUNDS OF THIS INVENTION

The major antimicrobial components of the antimicrobial complex 510 were isolated as individual components by passage of the antimicrobial complex 510 through a partition chromatography column, Sephadex LH-20 (Pharmecia), followed by elution with methanol, followed by droplet countercurrent chromatography (DCC) on a column using as the eluate a mixture of chloroform:methanol:water (7:13:8, v/v/v).

Preparative reverse phase chromatography on MCI CHP 20P gel (Mitsubishi) using a linear methanol gradient in acid on the antimicrobial complex 510 can also be used to obtain a separation of the three novel macrolactam monosaccharides of formula I(d) and of the known macrolactam monosaccharide of formula 4.

The compounds represented by formula I(d) and 4 were analyzed by high resolution Fast Atom Bombardment-Mass Spectral (FAB-MS) and carbon-13 NMR. The physicochemical data for the compounds represented by formulas 1 and 2 are shown in Tables IX, X and XI.

Table IX

| High Resolution FAB-Mass Spectral Data for the Compounds of Formulas I(d) and 4 | | | | |
|---|---|---|---|---|
| Compound | $MH^+$ | | | |
| | | Calc. | Found | Δ PPMm/z |
| Formula I(d) ($R_1 = R_2 = R_3 = H$) | 415.3172 | 415.3162 | 2 | 252,164,146 |
| Formula I(d) $R_1 = R_2 = H$, $R_3 = CH_3$ | 429.3328 | 429.3333 | 1 | 266,164,146 |
| Formula I(d) $R_1 = H$; $R_2 = R_3 = CH_3$ | 443.3485 | 443.3353 | 7 | 280,164,146 |
| Formula 4 | 457.3641 | 457.3674 | 3 | 294,164,146 |

Based on the high resolution FAB-MS data in Table IX, the molecular formulas for compounds of formulas I-(d) and 4 were determined and are given in Table X.

Table X

| Molecular Formulas of Compounds of Formulas I(d) and 4 | |
|---|---|
| Compound | Molecular Formula |
| Formula I(d) $R_1 = R_2 = R_3 = H$ | $C_{22}H_{42}N_2O_5$ |
| Formula I(d) $R_1 = R_2 = H$; $R_3 = CH_3$ | $C_{23}H_{44}N_2O_5$ |
| Formula I(d) $R_1 = H$; $R_2 = R_3 = CH_3$ | $C_{24}H_{46}N_2O_5$ |
| Formula 4 | $C_{25}H_{48}N_2O_5$ |

We determined that the C-13 NMR (fully decoupled and INEPT) spectra of the compounds of formula I-(d) and the C-13 NMR for the compound of formula 4; the data are listed in Table XI.

Table XI

| C-13 NMR Data for Compounds of Formulas I(d) and 4 | | | | |
|---|---|---|---|---|
| | Formula I(d) | | | |
| Carbon | $R_1 = R_2 = R_3 = H$ | $R_1 = R_2 = H$; $R_3 = CH_3$ | $R_1 = H$; $R_2 = R_3 = CH_3$ | Formula 4 |
| 2 | 38.9 | 39.1 | 39.8 | 39.2 |
| 3 | 28.5 | 28.8 | 28.3 | 28.1 |
| 4 | 29.9 | 29.6 | 22.4 | 25.5 |
| 5 | 36.1 | 36.4 | 42.0 | 41.2 |
| $CH_2$ 5′ | -- | -- | 22.0 | 21.5 |
| $CH_3$ 5″ | 17.3 | 17.4 | 9.2 | 8.9 |
| 6 | 79.5 | 79.9 | 78.3 | 76.9 |
| 7 | 20.4 | 20.6 | 22.0 | 21.7 |
| 8 | 25.8 | 26.0 | 26.2 | 22.7 |
| 9 | 31.1 | 29.9 | 28.6 | 39.0 |
| $CH_2$ 9′ | -- | -- | -- | 27.6 |
| $CH_3$ 9″ | 18.5 | 18.6 | 18.9 | 12.6 |
| 10 | 35.4 | 35.8 | 36.0 | 32.5 |
| 11 | 24.5 | 23.2 | 23.5 | 25.5 |
| 12 | 34.5 | 32.7 | 33.2 | 33.9 |
| 13 | 42.6 | 51.7 | 49.8 | 50.7 |
| $CH_2$ 13′ | -- | 27.6 | 26.2 | 26.9 |
| $CH_3$ 13″ | 13.8 | 12.6 | 12.9 | 12.3 |
| 14 | 178.6 | 178.9 | 179.0 | 178.2 |
| 1′ | 96.1 | 96.4 | 98.0 | 97.5 |
| 2′ | 70.7 | 70.5 | 70.4 | 72.9 |
| 3′ | 54.3 | 54.3 | 55.2 | 53.8 |
| 4′ | 69.3 | 69.6 | 70.7 | 71.0 |
| 5′ | 68.4 | 69.6 | 69.9 | 69.8 |
| 6′ | 17.2 | 17.3 | 17.7 | 17.7 |

The compound of formula 4 is shown below

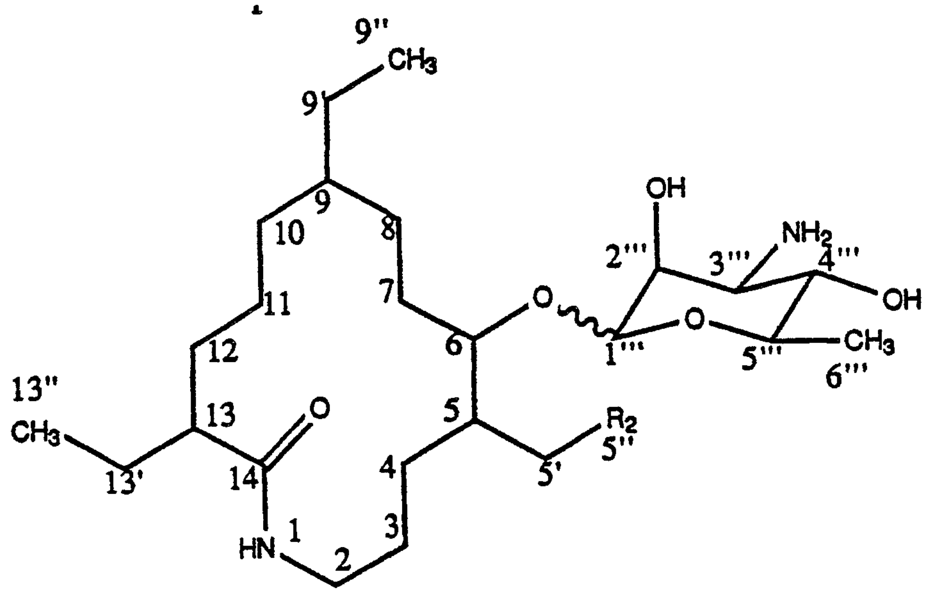

4

The physiochemical data in Tables IX, X and XI indicate the compounds of formulas I(d) and 4 are fourteen membered macrocyclic lactams and have the same amino sugar, mycosamine, and such data are consistent with the structures for the macrolactam monosaccharides represented by formula I(d).

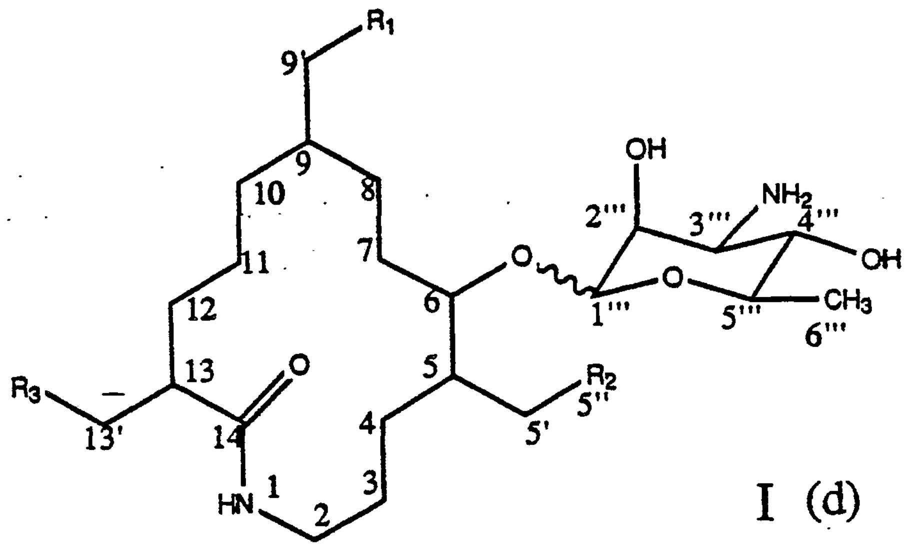

I (d)

$R_1 = R_2 = R_3 = H$; $R_1 = R_2 = H, R_3 = CH_3$; $R_1 = H, R_2 = R_3 = CH_3$

THE BIOLOGICAL ACTIVITY OF THE ANTIMICROBIAL COMPLEX, THE COMPOUND OF FORMULA I(d)

The antimicrobial complex 510 of this invention exhibits both antifungal activity and antibacterial activity in vitro against Gram positive and Gram negative microorganisms.

The three compounds represented by formula I(d), isolated from the antimcrobial complex 510, exhibit in vitro antifungal activity in a Sabouraud dextrose broth ("SDB") medium against seven species of Candida and six species of dermatophytes and antifungal activity against seven species of Candida in Eagles Minimum Essential Medium ("EMEM").

The in vitro antifungal data on the compounds of formula I(d) are presented in Table XII.

Table XII

| In vitro Antifungal Data on the Compounds of Formula I(d) | | | | |
|---|---|---|---|---|
| | | Compounds of | | |
| Formula I(d) | | | | |
| Organism (No. of Strains) | Medium | A | B | C |
| Candida (7) | SDB | 29 | 18 | 4.4 |
| Dermatophytes (6) | SDB | >114 | >128 | >57 |
| Candida (7) | EMEM | >105 | >71 | 24 |
| A $R_1 = R_2 = R_3 = H$ | | | | |
| B $R_1 = R_2 = H$; $R_3 = CH_3$ | | | | |
| C $R_3 = R_2 = CH_3$; $R_1 = H$ | | | | |

The following examples illustrate preparation of compounds of Formula I(d):

## EXAMPLE 4

### Preparation of the Antimicrobial Complex 510 of this Invention

#### A. Inoculum Preparation

1) Initial Stage

Prepare a 250 mL Erlemneyer flask with 70 mL of the following germination medium:

| | |
|---|---|
| Beef Extract | 3 g |
| Tryptone | 5 g |
| Yeast Extract | 5 g |
| Cerelose | 1 g |
| Potato Starch | 24 g |
| Calcium Carbonate | 2 g |
| Tap Water | 1000 mL |
| AF-1* | 1 mL |

*AF-1 is an antifoam agent available from Dow Corning Corp., Midland, MI 48641.

Sterilize the broth and after cooling, add 3.5 mL of a frozen whole broth sample of the microorganism of this invention from a previously prepared inoculum to each flask broth. Incubate at 30 °C with continual agitation at 300 rpm for 48 hours.

2) Second Stage

Transfer 25mL of the first stage germination broth to each of twenty 2-liter Erlenmeyer flasks, each

containing 500 mL of the same germination medium and which had been previously pH adjusted and sterilized. Incubate at 30° C with continual agitation at 300 rpm for 48 hours.

B. Fermentation

In a 150L fermentor, add 100L of the following medium:

| | g/L |
|---|---|
| Cotton Seed flour | 10.0 |
| Peptones | 1.0 |
| Consteep Liquor | 5.0 ml |
| Arabinose | 5.0 |
| Cerelose | 5.0 |
| $MgCl_2 \cdot 6H_2O$ | 0.5 |
| $ZnSO_4 \cdot 7H_2O$ (5% Solution) | 1.0 ml |
| $FeSO_4 \cdot 7H_2O$ (1.4% Solution) | 1.0 ml |
| Antifoam (AF-1 Dow Corning) | 1.0 ml |

Adjust the pH of the medium to 7.0 and add the inorganic salts and then sterilize the medium. Inoculate the fermentation medium with 5 volume % of the second stage inoculum preparation of Step A. Incubate the fermentation mixture at 30° C with 1.6 cubic feet per minute of air flow and 350 rpm agitation for about 90 hours. Use a 150L fermentor to produce about 130L of a whole broth containing antimicrobial complex 510.

C. Isolation

Option #1

Extract 310L of the whole fermentation broth at a pH of 9.5 produced in accordance with the fermentation procedure hereinabove with two 500L volumes of ethyl acetate. The ethyl acetate extracts were concentrated in vacuo to 10L and extracted with 5L of water at a pH of 2. The aqueous extracts were adjusted to pH 8.5 and were reextracted with 5L of n-butanol. The combined n-butanol extracts were concentrated to 1L and washed with 2L of water three times. Concentrate the washed n-butanol extract to 1L and dissolve the concentrate in 3L of methanol. Add to the so-formed methanol solution, 3.5L of diethylether:hexane (1:1, v/v) to produce 850 mg of crude antimicrobial complex 510 as a gum.

The crude antimicrobial complex 510 was purified by use of Sephadex LH 20 in methanol. Chromatography was carried out using a 3cm(diameter) x 60cm length column, eluting at 2.5ml/min, collecting five fractions. Fractions 24-45 were combined and concentrated in vacuo to yield 300mg pf crude cpmplex 510.

The compounds of formula I(d) were obtained as three substantially chemically pure compounds by use of droplet counter current chromatography using chloroform:methanol:water (7:13:8, v/v/v) mixture. The physiochemical data for the compounds of formula 1 are summarized in Tables IX, X and XI.

Option #2

The whole fermentation broth (300L) was adjusted to pH 2 with addition of acid, and stirring, then readjusted to pH 9.5 with addition of base. Extract 300L of the whole broth at a pH of 9.5 with two 500L volumes of ethyl acetate. The ethyl acetate extracts were concentrated in vacuo to approximately 10L and extracted with 5L of water at a pH of 2. The aqueous extracts were adjusted to pH 8.5 and reextracted with 5L of n-butanol. The combined n-butanol extracts were concentrated to approximately 1L, and then poured into 3.5L of diethyl ether with vigorous stirring. The crude antimirobial complex 510 (8g) precipitated as a gum.

The crude antimicrobial complex 510 was purified by chromatography on a MCI CHP20P gel (75-150 μ)

using a column (1-3/16" diameter and 48" length) eluting with a linear gradient of 30% aqueous methanol to 90% aqueous methanol in $H_2O$ adjusted to pH 2 with acetic acid. The flow rate was 2.5mL/min and 20mL fractions were collected. Fractions 210-224 contained the compound of formula I(d), ($R_1 = R_2 = R_3 = H$) (15mg), fraction 238-248 contained the compound of formula I(d), ($R_1 = R_2$,H, $R_3 = CH_3$) (60mg) and fractions 249-276 contained the compound of formula I(d), ($R_1 = H$, $R_2 = R_3 = CH_3$) (112mg).

## COMPOUNDS OF FORMULA I HAVING SUBSTITUENTS (e):

(hereinafter compound of formula I(e))

## THE MICROORGANISM

The microorganism used for the production of antimicrobial complex 515 and the compound of this invention is a biologically pure culture of Actinomadura vulgaris subsp. lanata subsp. nov. Shearer, Brodsky and Horan SCC 1777, ATCC 53715.

A viable culture of this microorganism has been deposited in the collection of the American Type Culture Collection (ATCC) in Rockville, Md., where it has been assigned accession number ATCC 53715. The deposit date is January 26, 1988.

The microorganism was isolated from a sample of soil collected in Missouri. It had been characterized and found to have the microscopic, macroscopic, and whole cell hydrolysis properties of the genus Actinomadura.

## DESCRIPTION OF THE PRODUCING STRAIN: ACTINOMADURA VULGARIS SUBSP LANATA SCC 1777, ATCC 53715

Source material for the following taxonomic evaluations was a frozen preparation of a pure culture of Actinomadura vulgaris subsp. lanata SCC 1777, ATCC 53715. Inoculum for the biochemical and physiological tests was prepared according to the procedures of Horan and Brodsky [Horan and Brodsky, Int. J. Syst. Bacteriol., Vol. 32, pp. 195-200 (1982)]. The incubation temperature for the biochemical and physiological tests was 30 °C. Readings of the results were made at various times up to 21 days for the plate media. Most of the tubed media were read at various times up to 28 days. The tests for decomposition of urea, allantoin and hippurate, as well as the tests for the reduction of nitrates were read for six weeks.

## MORPHOLOGY

Morphological observations of the producing strain of the microorganism of this invention were made on plates of water agar, AV-agar [Nonomura and Ohara, J. Ferment. Technol., Vol. 47, pp. 463-469 (1966)] or modified inorganic salts-starch agar [Difco inorganic salts-starch agar (ISP-4), 12 g; Difco Bacto agar, 15 g; distilled water, 800 ml; soil extract 200 ml; thiamine HCL, 0.5 mg; riboflavin, 0.5 mg; niacin, 0.5 mg; pyridoxine HCL, 0.5 mg; inositol, 0.5 mg; calcium pantothenate, 0.5 mg; p-aminobenzoic acid, 0.5 mg; biotin, 0.25 mg]. Plates were incubated at 30 °C and observed for 4 to 6 weeks.

A. vulgaris subsp. lanata is a gram positive, filamentous organism that forms a well-developed, moderately branching non-fragmenting substrate hyphae approximately 0.4 µm to 0.8 µm in diameter. Spores do not appear to be present. The aerial mycelia are approximately 0.3 µm to I.O µm in diameter and bear chains of 6 to 28 spores either sessible or most often borne on sporophores of varying lengths. The spore chains are straight, hooked, irregularly curved or arranged in spirals of 2 to 5 turns. The spirals are most often loosely coiled or may be tightly appressed. The spores are usually round to ovoid, 0.9 µm to 1.3 µm in diameter. The spore surface is irregularly folded. Motile elements are not formed in either the

substrate or aerial mycelia.

## CHEMOTAXONOMY

Purified cell wall preparations of the producing strain of this invention were analyzed by the method of Becker [Becker et. al., Appl. Microbiol., Vol. 12, pp. 421-423 (1964)] and shown to contain the meso isomer of 2,6-diaminopimelic acid, alanine, glutamic acid, glucosamine, muramic acid and a trace of mannose. Whole-cell hydroysates were analyzed by the method of Lechevalier [Lechevalier, M.P., J. Lab. Clin. Med., Vol. 71, pp. 934-944 (1968)] and shown to contain glucose, mannose, madurose, ribose, and a trace of rhamnose. The phospholipids present are diphosphatidylglycerol, phosphatidylinositol, phosphatidylinositol-mannosides, phosphatidylmethylethanolamine, phosphatidylethanolamine, and unknown glucosamine containing phospholipids. Thus, the producing strain of the microorganism of this invention has a type III cell wall with a type B whole-cell sugar pattern and a type P IV phospholipid composition [Lechevalier et. al., Biochem. System. Ecol., Vol. 5, pp. 249-260 (1977)], typical of actinomadurae.

## PHYSIOLOGICAL AND BIOCHEMICAL CHARACTERISTICS

The procedures used were those cited by Gordon [Gordon, R.E., J. Gen. Microbiol., Vol. 45, pp. 355-364 (1966)], Luedemann and Brodsky [Luedemann and Brodsky, "Antimicrob. Agents Chemother." pp47-52 (1965)] and Horan and Brodsky [Horan and Brodsky, Int. J. Syst. Bacteriol., Vol. 32, pp. 195-200 (1982)]. The producing strain of the A. vulgaris subsp. lanata, SCC 1777, produces acid from adonitol, D-arabinose, L-arabinose, D-fructose, L-fucose, D-galactose, glucose, glycerol, i-inositol, maltose, D-mannitol, D-mannose, α-D-melibiose, β-methyl-D-glucopyranoside, α-L-rhamnose, D-ribose, sucrose, D-trehalgose, D-sorbitol and D-xylose but not from dulcitol, i-erythritol, D-melizitose D-raffinose, or L-methyl-D-glucoside. Adenine, hypoxanthine, L-tyrosine, elastin, xylan, caesin and hippurate are hydrolyzed but guanine, xanthine and chitin are not. Gelatin is both hydrolyzed and liquified. Starch hydrolysis is negative. Urease and allantoinase are not formed. Nitrate is reduced to nitrite. Melanin and hydrogen sulfide are not formed. Growth does not occur at 10°C or at 45°C. Growth is fair at 42°C. The microorganism of this invention SCC 1777, grows poorly in a 4% NaCl solution. Acetate, formate, lactate, pyruvate and succinate are utilized; benzoate, butyrate and tartrate are not.

A. vulgaris subsp. lanata grows in the presence of 50 µg/ml of gentamicin, rifamycin, erythromycin, penicillin G, cephalothin and spectinomycin and in the presence of 10 mcg/ml gentamicin, sisomicin, streptomycin and neomycin. Growth is poor in the presence of 50 mcg/ml of sisomicin, neomycin, kanamycin, clindamycin and everninomicin.

## DESCRIPTION OF A. VULGARIS SUBSP. LANATA ON VARIOUS MEDIA

All plates were incubated at 30°C and observed at intervals up to 28 days. The common names for the colors were choosen after comparison with color chips from the ISCC-NBS centroid color charts, the "Color Harmony Manual", Ed. 4 (Container Corp. America, 1958), or the "Methuen Handbook of Color" (Eyre Methuen, London, 1981). On all media tested, the substrate mycelium of A. vulgaris subsp. lanata is cream to yellow-brown to brown. The aerial mycelium is white to ivory to yellowish-gray and frequently woolly in texture. The B-vitamins are not essential for growth but seem to exhance production of aerial mycelia and spores. Yellow-brown soluble pigments are produced. Characteristics are presented in Table I.

Based on the yellow-brown pigmentation of the substrate mycelia, spore morphology and physiological characteristics A. vulgaris subsp. lanata SCC 1777 (ATCC 53715) appears to be most closely related to A. vulgaris subsp. vulgaris ATCC 53748 (discussed previously) and A. spiralis IFO 14097.

In direct comparisons A. spiralis was found to form shorter spore chains (10 to 16 spores per chain); to hydrolyze starch; to form urease and allantoinase; to be susceptible to gentamicin, sisomicin and neomycin at 10 mcg/ml; not to hydrolyze xylan or produce acid from sorbitol or sucrose and not to exhibit antibiotic activity.

A. vulgaris subsp. lanata SCC 1777 is similar to A. vulgaris subsp. vulgaris ATCC 53748 in substrate

mycelial pigmentation, morphology of the spore bearing hyphae, biochemical and physiological characteristics and antibiotic production. They differ in growth temperature range, resistance to novobiocin and acid production from α-methyl-D-glucoside and D-raffinose. In addition, the macroscopic appearance of the aerial mycelia of A. vulgaris subsp. lanata SCC 1777 (ATCC 53715) is consistently wooly in texture when compared to A. vulgaris ATCC 53748. Based on these differences, ATCC 53715 is considered to be a distinct new subspecies of A. vulgaris designated A. vulgaris subsp. lanata.

The subspecies name selected (lanata L. adj. wool bearing, woolly) refers to the woolly texture of the aerial mycelia.

TABLE XIII

Macroscopic Appearance of Actinomadura vulgaris subsp. lanata SCC 1777, ATCC 53715 on various descriptive media[a]

| MEDIUM | | RESULT |
|---|---|---|
| Yeast Extract-Malt Extract Agar (ISP 2) | G: | good to excellent |
| | AM: | present; sparse, white |
| | SC: | sterile to few |
| | DFP: | absent |
| | SMP: | yellow-brown |
| Oatmeal Agar (ISP 3) | G: | fair to good |
| | AM: | absent |
| | SC: | absent |
| | DFP: | absent |
| | SMP: | yellow-brown |
| Inorganic Salts-Starch Agar (ISP 4) | G: | fair to good |
| | AM: | present; sparse to moderate, white to yellowish gray (Methuen, 4B2) |
| | SC: | numerous |
| | DFP: | absent |
| | SMP: | yellow-brown |
| Glycerol-Asparagine Agar (ISP 5) | G: | fair |
| | AM: | present; moderate, white |
| | SC: | sterile to moderate |
| | DFP: | absent |
| | SMP: | yellow-brown |
| Peptone-Yeast Extract-Iron Agar (ISP 6) | G: | fair to good |
| | AM: | absent |
| | SC: | absent |
| | DFP: | pale yellow-brown |
| | SMP: | yellow-brown |
| AV Agar | G: | fair |
| | AM: | present; abundant, yellowish-gray (Methuen 4B2) |
| | SC: | numerous |
| | DFP: | absent |
| | SMP: | yellow-brown |
| Gauze's Mineral Agar I | G: | fair |
| | AM: | absent |
| | SC: | absent |
| | DFP: | absent |
| | SMP: | yellow-brown |

TABLE XIII (continued)

| MEDIUM | RESULT |
|---|---|
| ATCC Medium 172 | G: excellent<br>AM: absent<br>SC: absent<br>DFP: present; pale yellow-brown<br>SMP: yellow-brown |
| Czapek-Sucrose Agar | G: fair<br>AM: present; sparse to moderate, white<br>SC: sterile<br>DFP: absent<br>SMP: yellow to yellow-brown |
| Glucose-Yeast Extract Agar | G: good to excellent<br>AM: absent<br>SC: absent<br>DFP: absent<br>SMP: yellow-brown |

[a] G = growth; AM = aerial mycelium; SC = spore chain; DFP = diffusible pigment; SMP = substrate mycelium pigmentation

## FERMENTATION OF THE MICROORGANISM

The antimicrobial complex 515 of this invention is produced when the elaborating microorganism, Actinomadura vulgaris subsp. lanata SCC 1777, ATCC 53715 is grown in an aqueous nutrient medium under submerged aerobic conditions at a temperature of about 27°C to about 40°C, preferably at from 27°C to 35°C, and at a pH of from about 6.5 to 8.0 with agitation until substantial antimicrobial activity is imparted to the medium. Temperature studies indicate that the organism grows rapidly at about 30°C. Therefore, the fermentation is preferably conducted employing a single temperature pattern of 30°C for a period of about 24 to about 96 hours preferably about 70 hours. The fermentation is generally conducted from about 3 to 7 days, preferably for about 3 days.

To determine when peak antimicrobial production has been reached, samples of the fermentation broth were assayed every 24 hours (starting at 48 hrs.) for antimicrobial content by bioassay of the whole broth against Staphylococcus aureus ATCC 209P (pH 8.0) Escherichia coli ATCC 10536 (pH 8.0) and Candida albicans Wisconsin. The growth of the organism (packed cell volume), pH and dissolved oxygen levels are determined either intermittantly or continuously.

As nutrient medium, there is employed any suitable medium containing a source of carbon, for example an assimilable carbohydrate, and a source of nitrogen, for example an assimilable nitrogenous or proteinaceous material and various mineral salts.

The medium employed for the fermentation contained NZ Amine A (an enzymatic hydrolysate of casein) and soluble starch as the major sources of nitrogen and carbon, respectively. Under these conditions, the microorganism, SCC 1777, produced antimicrobial complex 515 containing at least one biologically active component as determined by bioautography against both S. aureus, E. coli and C. albicans of the complex after development of a thin layer chromatography plate in 2:2:1 (v/v/v) chloroform: methanol, pH 3.5 acetate buffer.

The foregoing media are exemplary of the nutrients utilized by Actinomadura vulgaris subsp. lanata to produce antimicrobial complex 515. However, it is obvious to those trained in fermentation science that a wide range of nutrients obtained from a number of suppliers may be substituted for the foregoing, and that generally good growth and antibiotic production can be obtained, such nutrients being the functional

equivalent to those set forth herein.

The fermentation is generally conducted by initially sterilizing the fermentation medium prior to the addition of the inoculum.

The pH of the fermentation medium is generally maintained at from 6.5 to 8.0, a pH of from 6.5 to 7.5 being preferred. Prior to sterilization, the pH of the medium is usually adjusted to 6.7 and prior to inoculation, the pH is usually adjusted to 7.0.

The fermentation was initiated by addition of the inoculum to the broth. Generally, inoculum volume is 2.5% of total broth volume. The inoculum is prepared by addition of a sample of the frozen whole broth to an appropriate medium. A particularly preferred inoculum preparation medium comprises beef extract, 0.3%; tryptone, 0.5%; cerelose, 0.1% potato starch, 2.4%; yeast extract, 0.5%; and calcium carbonate, 0.2% (all percents by weight). The pH of the inoculum medium is adjusted to 7.5 prior to sterilization. The inoculum stage of the fermentation usually requires from 24 to 96 hours with 2 days for each of two stages being preferred and is generally conducted at about 30 °C with agitation. Agitation and a positive air flow, generally about 3.5 L/min. and a temperature of about 30 °C are employed during the fermentation. A particularly preferred fermentation medium comprises yeast extract 0.5%; NZ-Amine A, 0.5%; cerelose, 1.0%; soluble starch, 2.0%; calcium carbonate 0.4%; and 0.1 volume % of a $10^{-3}$ molar cobalt (II) chloride solution. The pH of the solution is adjusted to 7 prior to the addition of calcium carbonate. An antifoam agent such as Antifoam AF-1 (Dow Corning) is added, if necessary, to the fermentors to control foam.

## ISOLATION AND PURIFICATION OF THE ANTIMICROBIAL COMPLEX 515

The antimicrobial complex 515 of this invention contains a complex mixture of antimicrobials, including as the major component the compound represented by formula I(e), a macrolactam monosaccharide along with several minor components. The antimicrobial complex 515 of this invention is isolated by extraction of the whole fermentation broth with n-butanol. The n-butanol extract is washed, dried, concentrated, and dissolved in methanol; the so formed mixture is added to an ether:hexane mixture. The so-formed precipitate is the antimicrobial complex 515 of this invention which exhibits antifungal activity against *Candida* species and antibacterial activity against Gram positive and Gram negative bacteria.

## ISOLATION AND PURIFICATION OF THE COMPOUNDS OF THIS INVENTION

The major antimicrobial component of the antimicrobial complex 515 was obtained by preparative High Performance Liquid Chromatography (HPLC) of the antimicrobial complex on a silica gel column using as the eluate a mixture of dichloromethane:methanol: triethylamine (95:5:0.25, v/v/v).

The compound represented by formula I(e) is a white solid, basic in nature and gives a positive color reaction with ninhydrin. The compound is fairly soluble in methanol, dimethyl sulfoxide, sparingly soluble in chloroform, ethyl acetate and insoluble in water and also shows white lipid like spot on TLC plate when sprayed with water.

The physicochemical data for the compound represented by formula I(e) are shown in Tables XIV and XV.

TABLE XIV

| PHYSIOCHEMICAL DATA | |
|---|---|
| Opt. Rot : | $[\alpha]_D^{26}$ = -6.7 (DMSO, c 0.5) |
| UV λ max : | End Absorption |
| IR(KBr) : | 3430, 3340, 2940, 1645, 1550, 1460, 1055 $cm^{-1}$ |
| FAB MS : | 443(M+H), 298, 280, 164, 146 |
| PMR($CD_3OD$ + $CDCl_3$) δ : | 0.76 (d, $CH_3$), 0.80 (t, 2-$CH_3$) 1.18 (d, $CH_3$), 0.9-1.7 ($CH_2$ & CH) 1.92 (m, 1H), 2.95 (dd, 1H), 3.4 (m, 2H), 3.55 (m, 1H), 4.8 (d, 1H) 6.7 (b, 1H), |

Table XV

| CARBON-13 NMR Shifts[a] | |
|---|---|
| Carbon | PPM |
| 2 | 38.2 |
| 3 | 27.0 |
| 4 | 24.5 |
| 5 | 40.3 |
| 5′ | 19.5 |
| 5″ | 8.3 |
| 6 | 76.0 |
| 7 | 20.7 |
| 8 | 30.5 |
| 9 | 47.6 |
| 9′ | 6.7 |
| 10 | 33.5 |
| 11 | 24.3 |
| 12 | 32.8 |
| 13 | 49.8 |
| 13′ | 26.0 |
| 13″ | 11.5 |
| 14 | 176.8 |
| Sugar | |
| 1‴ | 97.1 |
| 2‴ | 72.6 |
| 3‴ | 52.0 |
| 4‴ | 71.4 |
| 5‴ | 67.2 |
| 6‴ | 16.0 |

[a]$CD_3OD/CDCl_3$

The compound of this invention is levorotatory with a specific rotation of $[\alpha]_D^{26}$ = -6.7 and has no UV absorption. FAB mass spectrum shows $(M+H)^+$ peak at 443; thus, the molecular wt. of the compound of formula I(e) is 442. The characteristic peaks in IR at 1640 and 1550 $cm^{-1}$ indicate the presence of an amide. The physicochemical data of Tables XIV and XV is consistent with a macrolactam monosaccharide represented by the formula I(e).

## THE BIOLOGICAL ACTIVITY OF THE ANTIMICROBIAL COMPLEX, THE COMPOUND OF FORMULA I(e)

The antimicrobial complex 515 of this invention exhibits both antifungal activity and antibacterial activity *in vitro* against Gram positive and Gram negative microorganisms.

The compound represented by formula I(e), isolated from the antimicrobial complex 515, exhibits *in vitro* antifungal in a Sabouraud dextrose broth medium against seven species of *Candida* (geometric mean MIC of 0.91 mcg/mL) and six species of dermatophytes (geometric mean MIC of 80.6 mcg/ml) and three species of *Cryptococcus* (geometric mean MIC is 0.8 mcg/mL) and antifugal activity against seven species of *Candida* (geometric mean MIC is 3.62 mcg/mL) in Eagles Minimum Essential Medium.

The following example illustrates preparation of the compound of formula I(e):

## EXAMPLE 5

## Preparation of the Antimicrobial Complex 530 of This Invention

### A. Inoculum Preparation

#### 1) Initial Stage

Prepare a 250 mL Erlemneyer flask with 70 mL of the following germination medium:

| | |
|---|---|
| Beef Extract | 3 g |
| Tryptone | 5 g |
| Yeast Extract | 5 g |
| Cerelose | 1 g |
| Potato Starch | 24 g |
| Calcium Carbonate | 2 g |
| Tap Water | 1000 mL |
| AF-1* | 1 mL |

*AF-1 is an antifoam agent available from Dow Corning Corp., Midland, MI 48641.

Adjust the pH of the germination broth to 7.5. Sterilize the broth and after cooling, add 3.5 mL of a frozen whole broth sample of the microorganism of this invention from a previously prepared inoculum to each flask broth. Incubate at 30° C with continual agitation at 300 rpm for 48 hours.

#### 2) Second Stage

Transfer 25mL of the first stage germination broth to each of twenty 2-liter Erlenmeyer flasks, each containing 500 mL of the same germination medium and which had been previously pH adjusted and sterilized. Incubate at 30° C with continual agitation at 300 rpm for 48 hours.

### B. Fermentation

In a 2L fermentor, add 350 ml of the following medium:

| | |
|---|---|
| Yeast Extract | 5.0 g |
| NZ-Amine-A | 5.0 g |
| Cerelose | 10.0 g |
| Soluble Starch | 20.0 g |
| $CaCO_3$ 4.0 g | |
| Tap $H_2O$ | 1000.0 mL |
| $COCl_2 10^{-3}M$ | 1.0 mL |
| Antifoam (AF-1 Dow Corning) | 1.0 mL |

Adjust the pH of the medium to 7.0 and add the calcium carbonate and then sterilize the medium. After sterilization, adjust the pH of the medium to 7.0 with a sterile acidic solution. Inoculate the fermentation medium with 25 mL of the second stage inoculum preparation of Step A. Incubate the fermentation mixture at 30° C with 0.45 VVM (4.5 L/min) of air flcw and 350 rpm agitation for about 72 hours. Use a 150L fermentor to produce about 130L of whole broth containing antimicrobial complex 515.

C. Isolation

Extract 130L of the whole fermentation broth produced in accordance with the fermentation procedure hereinabove with two 50L volumes of n-butanol. Concentrate the combined n-butanol extracts to 1L and wash the 1L concentrate three times with 2L of water. Concentrate the washed n-butanol extracts to dryness and dissolve the concentrate in 200 mL of methanol: chloroform (1:1, v/v)

Add to the so-formed methanol solution, 3.5L of diethylether:hexane (1:1, v/v) to produce 18.4g of crude antimicrobial complex 515 as gum.

Purify the crude antimicrobial complex 515 on preparative HPLC (Water Associates, Preparative 500) using silica gel cartridges and as eluent dichloromethane:methanol:triethylamine (95:5:0.25, v/v/v) to produce 3.5g of the compound of formula I(e) as a white solid. The white solid is substantially chemically pure, basic in nature, gives a positive color reaction with ninhydrin, is fairly soluble in methanol, dimethylsulfoxide, is sparingly soluble in chloroform, ethyl acetate and insoluble in water. The physicochemical data for the compound of formula I(e) are summarized in Tables XIV and XV.

## MEDICAL USAGE OF THE COMPOUNDS OF THIS INVENTION

This invention also contemplates antimicrobially effective pharmaceutical compositions comprising an antimicrobially effective amount of a compound of formula 1 or pharmaceutically acceptable salts thereof in admixture with a pharmaceutically acceptable, non-toxic carrier adapted for topical, oral or parenteral use.

The preferred pharmaceutically acceptable salts are nontoxic acid addition salts formed by adding to the compounds of the present inventin about a stoichiometric amount of a mineral acid, such as HC1, HBr, $H_2SO_4$ or $H_3PO_4$, or of an organic acid, such as acetic, propionic, valeric, oleic, palmitic, stearic, lauric, benzoic, lactic, para-toluene sulfonic, methane sulfonic, citric, maleic, fumaric, succinic and the like.

The topical, oral and parenteral dosage forms may be prepared according to procedures well known in the art, and may contain a variety of ingredients.

In the case of topical formulations, e.g., ointments, creams, lotions, powders, tablets, pessaries or sprays, the formulation will contain about 0.1 to 10 grams of a compound of formula 1 per 100 grams of carrier.

Oral dosage forms include tablets, capsules, elixirs, suspensions, and the like. Tablets contain such excipients as starch or lactose; liquid forms may contain coloring of flavoring agents.

Parenteral forms to be injected intravenously, intramuscularly, or subcutaneously are usually in the form of a sterile solution, and may contain salts or glucose to make the solution isotonic.

In general, the dosage of compound of formula 1 administered to combat a given microbial infection is similar to the dosage requirements of the present commercial products miconazole, clotrimazole, and ketoconazole.

In general, the topical dosage range of the compound of formula 1 is from about 0.1% to about 10% by weight of a particular pharmaceutical composition formulated in single or divided doses, with the preferred range being about 0.5% to about 4% and with the most preferred range being about 1% to about 2%.

In general, the oral dosage for humans of the compound of formula 1 administered to combat a given microbial infection ranges from about 1 mg per kilogram of body weight to about 50 mg per kilogram of body weight per day, in single or divided doses, with about 2 mg per kilogram of body weight to about 20 mg per kilogram of body weight per day being preferred.

In general, the parenteral dosage for humans of the compound of formula 1 administered to combat a given microbial infection ranges for about 0.1 mg per kilogram of body weight per day, to about 20 mg per kilogram of body weight per day, in single or divided doses, with about 1 mg per kilogram of body weight per day being preferred.

It will be appreciated that the actual preferred dosages of the compounds of this invention or pharmaceutically acceptable salts thereof will vary according to the particular compound chosen, the particular composition formulated, the mode of application and the particular situs, host and disease being treated. Many factors that modify the action of the drug will be taken into account by the attending clinician, e.g. age, body weight, sex, diet, time of administration, rate of excretion, condition of the host, drug combinations, reaction sensitivities and severity of the disease. Administration can be carried out continuously or periodically within the maximum tolerated dose. Optimal application rates for a given set of conditions can be readily ascertained by the attending clinician using conventional dosage determination tests.

## Claims

1. A compound having the formula:

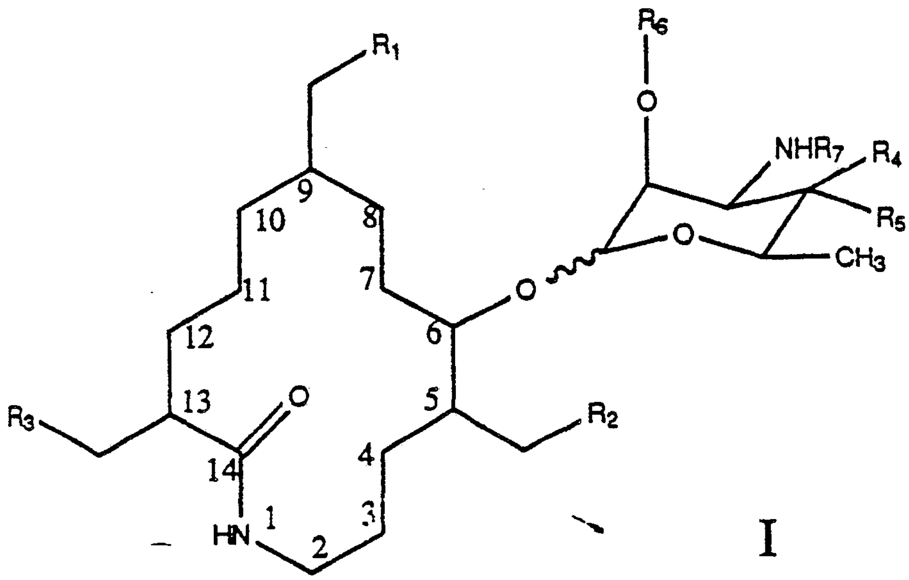

I

wherein either

(a) $R_1$, $R_2$, and $R_3$ are methyl,
$R_4$ is hydrogen,
$R_5$ is hydroxy,
$R_6$ is

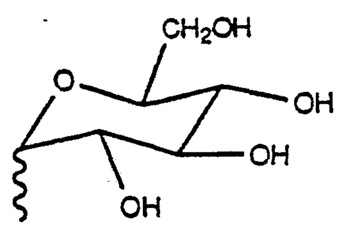

;

and
$R_7$ is hydrogen;
<u>or</u>

(b) $R_1$, $R_2$, and $R_3$ are methyl
$R_4$ is hydrogen,
$R_6$ is

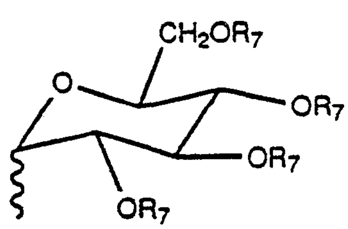

;

and

$R_5$ and each $R_7$ are independently a straight or branched chain ($C_1$-$C_{22}$) alkanoyl group, a straight or branched chain ($C_3$-$C_{22}$) alkenoyl group or hydrogen, with the proviso that $R_5$ or at least one $R_7$ is not hydrogen;

or

(c) $R_1$, $R_2$, and $R_3$ are methyl,
$R_4$ is hydroxy, and
$R_5$, $R_6$, $R_7$ are hydrogen;

or

(d) $R_1$, $R_2$, and $R_3$ are hydrogen, or
$R_1$ and $R_2$ are hydrogen and $R_3$ is methyl,
or $R_1$ is hydrogen and $R_2$ and $R_3$ are methyl,
$R_5$ is hydroxy, and
$R_4$, $R_6$ and $R_7$ are hydrogen;

or

(e) $R_1$ is hydrogen,
$R_2$ and $R_3$ are methyl
$R_4$ is hydroxy, and
$R_5$, $R_6$, and $R_7$ are hydrogen
or a pharmaceutically acceptable salt thereof.

2. A pharmaceutical composition comprising an antimicrobially effective amount of the compound of claim 1 and a pharmaceutically acceptable carrier or diluent.

3. A method for making a pharmaceutical composition for treating microbial infection comprising admixing a compound of claim 1 with a pharmaceutically acceptable carrier or diluent.

4. The compond of claim 1 wherein
$R_1$, $R_2$, and $R_3$ are methyl,
$R_4$ is hydrogen,
$R_5$ is hydroxy,
$R_6$ is

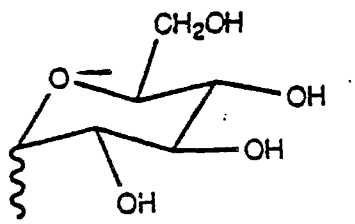

,

and
$R_7$ is hydrogen.

5. The compound of claim 1 wherein
$R_1$, $R_2$ and $R_3$ are methyl,
$R_4$ is hydrogen,
$R_6$ is

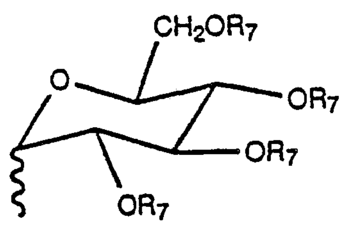

,

and
$R_5$ and each $R_7$ is independently a straight or branched chain ($C_1$-$C_{22}$) alkanoyl group, a straight or branched chain ($C_3$-$C_{22}$) alkenoyl group or hydrogen, with the proviso that $R_5$ or at least one $R_7$ is not hydrogen.

6. The compound of claim 1 wherein
$R_1$, $R_2$, and $R_3$ are methyl
$R_4$ is hydroxy, and
$R_5$, $R_6$, and $R_7$ are hydrogen;

7. The compound of claim 1 wherein
$R_1$, $R_2$, and $R_3$ are hydrogen, or
$R_1$ and $R_2$ are hydrogen and $R_3$ is methyl, or
$R_1$ is hydrogen and $R_2$ and $R_3$ are methyl,
$R_5$ is hydroxy, and
$R_4$, $R_6$, and $R_7$ are hydrogen;

7. The compound of claim 1 wherein
$R_1$ is hydrogen
$R_2$ and $R_3$ are methyl
$R_4$ is hydroxy, and
$R_5$, $R_6$, and $R_7$ are hydrogen.

9. A biologically pure culture of the microorganism Actinomadura fulva subsp. indica, SCC 1840 having the identifying characteristics of ATCC 53714.

10. Antimicrobial complex 530 produced by cultivating a strain of Actinomadura fulva subsp. indica having the identifying characteristics of ATCC 53714.

11. A biologically pure culture of the microorganism Actinomadura vulgaris subsp. vulgaris, SCC 1776 having the identifying characteristics of ATCC 53748.

12. Antimicrobial complex 517 produced by cultivating a strain of Actinomadura vulgaris subsp. vulgaris having the identifying characteristics of ATCC 53748.

13. A biologically pure culture of the microorganism Actinomadura fulva subsp. uruguayensis SCC 1778 having the identifying characteristics of ATCC 53713.

14. Antimicrobial complex 510 produced by cultivating a strain of Actinomadura fulva subsp. uruguayensis having the identifying characteristics of ATCC 53713.

15. A biologically pure culture of the microorganism Actinomadura vulgaris subsp. lanata, SCC 1777 having the identifying characteristics of ATCC 53715.

16. Antimicrobial complex 515 produced by cultivating a strain of Actinomadura vulgaris subsp. lanata having the identifying characteristics of ATCC 53715.

European Patent Office

# EUROPEAN SEARCH REPORT

Application Number

EP 89 30 7708

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| X | CHEMICAL ABSTRACTS, vol. 89, no. 15, 9th October 1978, page 452, abstract no. 127757v, Columbus, Ohio, US; & JP-A-78 28 101 (DAINIPPON PHARMACEUTICAL CO., LTD) 16-03-1978<br>* Abstract *<br>----- | 1-16 | C 07 H 17/02<br>A 61 K 31/70<br>C 12 P 19/60 //<br>(C 12 P 19/60<br>C 12 R 1:03 ) |
| | | | TECHNICAL FIELDS SEARCHED (Int. Cl.5) |
| | | | C 07 H 17/00<br>A 61 K 31/00<br>C 12 P 19/00<br>C 12 R 1/00 |

The present search report has been drawn up for all claims

| Place of search | Date of completion of the search | Examiner |
|---|---|---|
| THE HAGUE | 03-11-1989 | VERHULST W. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document